# EUROPEAN PATENT APPLICATION

(11) **EP 4 545 509 A1**
(43) Date of publication of application: **30.04.2025**
(21) Application number: 22948058.7
(22) Date of filing: 13.12.2022
(51) Int. Cl.: C07C 7/04, C07C 4/04, C07C 6/12, C07C 15/04, C07C 15/06, C07C 15/08, C10G 9/36

(54) **METHODS FOR PRODUCING MONOCYCLIC AROMATIC HYDROCARBONS, TEREPHTHALIC ACID, AND POLYETHYLENE TEREPHTHALATE, AND METHODS FOR MANAGING SAME**

(30) Priority: 21.06.2022 JP 2022099930
(71) Applicant: ENEOS Corporation, Chiyoda-ku Tokyo 100-8162 (JP); Mitsubishi Corporation, Tokyo, 100-8086 (JP)
(72) Inventor: SODA, Tadakatsu, Tokyo 100-8162 (JP); ONO, Junichiro, Tokyo 100-8086 (JP); SATO, Kota, Tokyo 100-8162 (JP)
(74) Representative: Müller-Boré & Partner Patentanwälte PartG mbB
(86) International application number: PCT/JP2022/045893
(87) International publication number: WO 2023/248499

(57) **Abstract**

[Problem] To provide a method capable of producing a monocyclic aromatic hydrocarbon such as benzene or xylene effective as a base raw material, with a naphtha raw material containing renewable naphtha.

[Solution] A method for producing at least one monocyclic aromatic hydrocarbon of benzene or xylene with a naphtha raw material containing renewable naphtha, the method including step (A-1) of pyrolyzing the naphtha raw material in the presence of water vapor, to produce and separate toluene, and step (A-2) of subjecting toluene to disproportionation reaction or transalkylation reaction, to produce and separate at least one monocyclic aromatic hydrocarbon of benzene or xylene.

## Description

### BACKGROUND OF THE INVENTION

### Field of the Invention

The present invention relates to methods for producing monocyclic aromatic hydrocarbon, terephthalic acid (including high-purity terephthalic acid), and polyethylene terephthalate (hereinafter, sometimes referred to as "PET"). The present invention also relates to methods for producing monocyclic aromatic hydrocarbon and terephthalic acid, and a management method for polyethylene terephthalate.

### Background Art

Benzene is a base raw material for styrene monomers, phenol, cyclohexane, or the like, and is processed into chemical products such as plastics, synthetic rubber or nylon and utilized in various situations of daily commodities such as plastic products or clothing items. Para-xylene is also a base raw material for terephthalic acid, and is processed into polyester fibers or the like and utilized in various situations of daily commodities.

Such useful base raw materials such as benzene and para-xylene are increasingly demanded.

Meanwhile, according to a current decarbonization trend, the movement for green raw materials is rapidly being spread also in the petrochemical industry. There are actively developed renewable raw material-derived base raw materials and methods involving processing such base raw materials to produce renewable raw material-derived chemical products, by partial or full replacement of petroleum-derived raw materials as raw materials of chemical products with biomass raw materials (for example, biological oils and fats such as waste edible oil and tall oil (by-product of pulp and paper).) or recycled raw materials, from the viewpoint of preservation of petroleum resources and reduction of carbon-dioxide emissions for sustainable development.

A method is disclosed in which petroleum-derived naphtha is used together with bio-naphtha, and pyrolyzed in the presence of water vapor, to produce propane and propene (see, for example, Patent Literature 1).

While such a method for producing propane and propene, described in Patent Literature 1, is known as a method for producing a renewable raw material-derived base raw material, no method for producing renewable raw material-derived benzene and/or para-xylene is known.

### Citation List

### Patent Literature

Patent Literature 1: JP-A 2016-501124

### SUMMARY OF THE INVENTION

### Technical Problem

Bio-naphtha has a compositional ratio close to that of a light fraction of petroleum-derived naphtha, and can be pyrolyzed in the presence of water vapor.

While bio-naphtha produces xylene when used as a raw material and pyrolyzed as described above, bio-naphtha is not suitable as a raw material for production of para-xylene because the content ratio of ethylbenzene as a by-product is high.

On the other hand, para-xylene is produced by hydrodesulfurizing heavy naphtha obtained from separation by distillation of crude oil, and thereafter isomerizing ortho-xylene and meta-xylene obtained by catalytic reforming reaction.

Thus, heavy naphtha is commonly used as a raw material of catalytic reforming reaction, and light naphtha or bio-naphtha having a compositional ratio close to that of light naphtha is not generally used as a raw material of catalytic reforming reaction.

In other words, an existing chemical industrial process conventionally known has not been a method capable of producing benzene or para-xylene effective as a base raw material (hereinafter, benzene and para-xylene are also collectively referred to as "monocyclic aromatic hydrocarbon") with bio-naphtha, and has not been able to provide any bio-naphtha-derived monocyclic aromatic hydrocarbon.

An object of the present invention is to provide a method capable of producing a monocyclic aromatic hydrocarbon such as benzene or para-xylene effective as a base raw material, with a naphtha raw material containing renewable naphtha.

Another object of the present invention is to provide a management method for a monocyclic aromatic hydrocarbon, usable in the production of a monocyclic aromatic hydrocarbon with a naphtha raw material containing renewable naphtha, in which the method involves assigning a value as a renewable product, to a monocyclic aromatic hydrocarbon product, depending on the content rate of the renewable naphtha contained in the naphtha raw material.

### Solution to Problem

The present inventors have made studies in order to solve the above problems, and as a result, have found that reaction steps usually not combined in any existing chemical industrial process are combined to provide a novel chemical industrial process and such a novel chemical industrial process can solve the above problems, and have completed the present invention having the following gist.

Specifically, the present invention encompasses the following.
[1] A method for producing at least one monocyclic aromatic hydrocarbon of benzene or xylene with a naphtha raw material containing renewable naphtha, the method including:
   step (A-1) of pyrolyzing the naphtha raw material in the presence of water vapor, to produce and separate toluene; and
   step (A-2) of subjecting toluene to disproportionation reaction or transalkylation reaction, to produce and separate at least one monocyclic aromatic hydrocarbon of benzene or xylene.
[2] The production method according to [1], wherein toluene is produced and separated by distillation or extraction of a component containing a monocyclic aromatic hydrocarbon, in a pyrolysis product obtained by pyrolyzing the naphtha raw material in the presence of water vapor, in the step (A-1).
[3] The production method according to [1] or [2], further including step (A-3) of subjecting xylene obtained in the step (A-2), to adsorption separation or crystallization separation, to separate para-xylene.
[4] The production method according to [3], including a step of subjecting a residue containing at least one of ortho-xylene or meta-xylene after separation of para-xylene in the step (A-3), to isomerization treatment, to produce para-xylene, and subsequently separating the para-xylene by adsorption separation or crystallization separation.
[5] The production method according to any of [1] to [4], wherein the monocyclic aromatic hydrocarbon contains a radioactive carbon atom ¹⁴C.
[6] Xylene containing a radioactive carbon atom ¹⁴C.
[7] The xylene according to [6], wherein the radioactive carbon atom ¹⁴C is derived from bio-naphtha.
[8] A method for producing terephthalic acid with a naphtha raw material containing renewable naphtha, the method including:
   step (A-3) of obtaining para-xylene by the method according to [3] or [4]; and
   step (A-4) of oxidizing the para-xylene, to obtain terephthalic acid.
[9] Terephthalic acid containing a radioactive carbon atom ¹⁴C.
[10] The terephthalic acid according to [9], wherein the radioactive carbon atom ¹⁴C is derived from bio-naphtha.
[11] A method for producing polyethylene terephthalate with a naphtha raw material containing renewable naphtha, the method including:
   step (A-4) of obtaining terephthalic acid by the method according to [8]; and
   step (A-5) of obtaining polyethylene terephthalate by condensation reaction of terephthalic acid and ethylene glycol.
[12] Polyethylene terephthalate containing a bio-naphtha-derived radioactive carbon atom ¹⁴C.
[13] A management method for a monocyclic aromatic hydrocarbon to be used in production of at least one monocyclic aromatic hydrocarbon of benzene or xylene with a naphtha raw material containing renewable naphtha, wherein
   the management method is a method for assigning a value as a renewable product, to the at least one monocyclic aromatic hydrocarbon, depending on a content rate of the renewable naphtha contained in the naphtha raw material,
   the management method includes at least one or more steps selected from step (V) of confirming that xylene and/or benzene are/is produced and step (W) of confirming that para-xylene is obtained,
   the step (V) includes the following step (V-1), the following step (V-2), and the following step (V-3),
   the step (V-1) is a step of confirming that toluene is produced from the naphtha raw material loaded to an apparatus for pyrolysis in the presence of water vapor (steam cracker),
   the step (V-2) is a step of confirming that xylene and/or benzene are/is produced from toluene loaded to a disproportionation apparatus, or loaded to a transalkylation (TA) apparatus taken together with a C9-based component,
   the step (V-3) is a step of confirming that xylene and/or benzene are/is produced from the naphtha raw material by treatment in the apparatus for pyrolysis in the presence of water vapor (steam cracker), and the disproportionation apparatus or the transalkylation (TA) apparatus in the listed order,
   the step (W) includes the following step (V-1), the following step (V-2), the following step (W-3), and the following step (W-4),
   the step (V-1) is a step of confirming that toluene is produced from the naphtha raw material loaded to an apparatus for pyrolysis in the presence of water vapor (steam cracker),
   the step (V-2) is a step of confirming that xylene and/or benzene are/is produced from toluene loaded to a disproportionation apparatus, or loaded to a transalkylation (TA) apparatus taken together with a C9-based component,
   the step (W-3) is a step of confirming that para-xylene is obtained from xylene loaded to a para-xylene apparatus,
   the step (W-4) is a step of confirming that para-xylene is obtained from the naphtha raw material by treatment in the apparatus for pyrolysis in the presence of water vapor (steam cracker), the disproportionation apparatus or the transalkylation (TA) apparatus, and the para-xylene apparatus in the listed order,
   the management method includes step (Z) of confirming a proportion of a product to which a value as a renewable product is to be assigned, in a case where the management method includes the step (V) and the step (W),
   the step (Z) includes the following step (Z-1), the following step (Z-2), the following step (Z-3), and the following step (Z-4),
   the step (Z-1) is a step of selecting one or more products to be assigned to renewable product(s), in products obtained by the disproportionation apparatus or the transalkylation (TA) apparatus, and the para-xylene apparatus,
   the step (Z-2) is a step of determining a value of a proportion (P) of the product(s) to be assigned to renewable product(s) in a proportion of the product(s) selected in the step (Z-1), with respect to the products obtained by the disproportionation apparatus or the transalkylation (TA) apparatus, and the para-xylene apparatus,
   the step (Z-3) is a step of grasping a value of a content rate (Q) of the renewable naphtha contained in the naphtha raw material, and
   the step (Z-4) is a step of comparing a value of the proportion (P) and a value of the content rate (Q), and confirming that the value of the proportion (P) is equal to or less than the value of the content rate (Q).
[14] The management method according to [13], wherein, in a case where the management method includes the step (V) and does not include the step (W), the management method includes step (X) of confirming a proportion of a product to which a value as a renewable product is to be assigned,
   the step (X) includes the following step (X-1), the following step (X-2), the following step (X-3), and the following step (X-4),
   the step (X-1) is a step of selecting one or more products to be assigned to renewable product(s), in products produced by the disproportionation apparatus or the transalkylation (TA) apparatus,
   the step (X-2) is a step of determining a value of a proportion (P) of the product(s) to be assigned to renewable product(s) in a proportion of the products selected in the step (X-1), with respect to the products obtained by the disproportionation apparatus or the transalkylation (TA) apparatus,
   the step (X-3) is a step of grasping a value of a content rate (Q) of the renewable naphtha contained in the naphtha raw material, and
   the step (X-4) is a step of comparing a value of the proportion (P) and a value of the content rate (Q), and confirming that the value of the proportion (P) is equal to or less than the value of the content rate (Q).
[15] The management method according to [13], wherein, in a case where the management method includes the step (W) and does not include the step (V), the management method includes step (Y) of confirming a proportion of a product to which a value as a renewable product is to be assigned,
   the step (Y) includes the following step (Y-1), the following step (Y-2), the following step (Y-3), and the following step (Y-4),
   the step (Y-1) is a step of selecting one or more products to be assigned to renewable product(s), in products produced by the para-xylene apparatus,
   the step (Y-2) is a step of determining a value of a proportion (P) of the product(s) to be assigned to renewable product(s) in a proportion of the product(s) selected in the step (Y-1), with respect to the products obtained by the para-xylene apparatus,
   the step (Y-3) is a step of grasping a value of a content rate (Q) of the renewable naphtha contained in the naphtha raw material, and
   the step (Y-4) is a step of comparing a value of the proportion (P) and a value of the content rate (Q), and confirming that the value of the proportion (P) is equal to or less than the value of the content rate (Q).
[16] The management method according to any of [13] to [15], wherein, in a case where two or more of the products to be assigned to renewable products are selected in the step (X-1), the step (Y-1) or the step (Z-1), the value of the proportion (P) of the products to be assigned to renewable products, determined in the step (X-2), the step (Y-2), or the step (Z-2) is a total value of respective proportions to be assinged to the two or more of the products selected.
[17] The management method according to [13], wherein, in a case where the management method includes the step (V) and the step (W), and two of the products to be assigned to renewable products, benzene and para-xylene, are selected in the step (Z-1), and
   the value of the proportion (P) of the products to be assigned to renewable products, determined in the step (Z-2), is a total value of a proportion (P1) to be assigned to renewable benzene, in a proportion of benzene relative to the products obtained by the disproportionation apparatus or the transalkylation (TA) apparatus, and the para-xylene apparatus, and a proportion (P2) to be assigned to renewable para-xylene, in a proportion of para-xylene with respect to the products obtained by the disproportionation apparatus or the transalkylation (TA) apparatus, and the para-xylene apparatus.
[18] A management method for terephthalic acid to be used in production of terephthalic acid with a naphtha raw material containing renewable naphtha, wherein
   the management method is a method for assigning a value as a renewable product, to the terephthalic acid, depending on a content rate of the renewable naphtha contained in the naphtha raw material,
   the management method includes step (E) of confirming that the terephthalic acid is obtained,
   the step (E) includes the following step (V-1), the following step (G-2), the following step (W-3), the following step (E-4), and the following step (E-5),
   the step (V-1) is a step of confirming that toluene is produced from the naphtha raw material loaded to an apparatus for pyrolysis in the presence of water vapor (steam cracker),
   the step (G-2) is a step of confirming that xylene is produced from toluene loaded to a disproportionation apparatus, or loaded to a transalkylation (TA) apparatus taken together with a C9-based component,
   the step (W-3) is a step of confirming that para-xylene is obtained from xylene loaded to a para-xylene apparatus,
   the step (E-4) is a step of confirming that terephthalic acid is obtained from para-xylene loaded to a terephthalic acid apparatus,
   the step (E-5) is a step of confirming that terephthalic acid is obtained from the naphtha raw material by treatment in the apparatus for pyrolysis in the presence of water vapor (steam cracker), the disproportionation apparatus or the transalkylation (TA) apparatus, the para-xylene apparatus, and the terephthalic acid apparatus in the listed order,
   the management method includes step (H) of confirming a proportion of a product to which a value as a renewable product is to be assigned,
   the step (H) includes the following step (H-1), the following step (H-2), the following step (H-3), and the following step (H-4),
   the step (H-1) is a step of selecting a product to be assigned to a renewable product, in products obtained by the terephthalic acid apparatus,
   the step (H-2) is a step of determining a value of a proportion (P) of the product to be assigned to a renewable product in a proportion of the product selected in the step (H-1), with respect to the products obtained by the terephthalic acid apparatus,
   the step (H-3) is a step of grasping a value of a content rate (Q) of the renewable naphtha contained in the naphtha raw material, and
   the step (H-4) is a step of comparing a value of the proportion (P) and a value of the content rate (Q), and confirming that the value of the proportion (P) is equal to or less than the value of the content rate (Q).
[19] A management method for polyethylene terephthalate to be used in production of polyethylene terephthalate with a naphtha raw material containing renewable naphtha, wherein
   the management method is a method for assiging a value as a renewable product, to the polyethylene terephthalate, depending on a content rate of the renewable naphtha contained in the naphtha raw material,
   the management method includes step (F) of confirming that polyethylene terephthalate is obtained,
   the step (F) includes the following step (V-1), the following step (G-2), the following step (W-3), the following step (E-4), the following step (F-5), and the following step (F-6),
   the step (V-1) is a step of confirming that toluene is produced from the naphtha raw material loaded to an apparatus for pyrolysis in the presence of water vapor (steam cracker),
   the step (G-2) is a step of confirming that xylene is produced from toluene loaded to a disproportionation apparatus, or loaded to a transalkylation (TA) apparatus taken together with a C9-based component,
   the step (W-3) is a step of confirming that para-xylene is obtained from xylene loaded to a para-xylene apparatus,
   the step (E-4) is a step of confirming that terephthalic acid is obtained from para-xylene loaded to a terephthalic acid apparatus,
   the step (F-5) is a step of confirming that polyethylene terephthalate is obtained from terephthalic acid loaded to a PET apparatus,
   the step (F-6) is a step of confirming that polyethylene terephthalate is obtained from the naphtha raw material by treatment in the apparatus for pyrolysis in the presence of water vapor (steam cracker), the disproportionation apparatus or the transalkylation (TA) apparatus, the para-xylene apparatus, the terephthalic acid apparatus, and the PET apparatus in the listed order,
   the management method includes step (J) of confirming a proportion of a product to which a value as a renewable product is to be assigned,
   the step (J) includes the following step (J-1), the following step (J-2), the following step (J-3), and the following step (J-4),
   the step (J-1) is a step of selecting a product to be assigned to a renewable product, in products obtained by the PET apparatus,
   the step (J-2) is a step of determining a value of a proportion (P) of the product to be assigned to a renewable product in a proportion of the product selected in the step (J-1), with respect to the products obtained by the PET apparatus,
   the step (J-3) is a step of grasping a value of a content rate (Q) of the renewable naphtha contained in the naphtha raw material, and
   the step (J-4) is a step of comparing a value of the proportion (P) and a value of the content rate (Q), and confirming that the value of the proportion (P) is equal to or less than the value of the content rate (Q).
[20] A management apparatus including a computer-readable storage medium in which a management program is stored, wherein
   the management apparatus implements the management program, to implement the management method according to any of [13] to [19].
[21] The management apparatus according to [20], wherein, after the management method is implemented, a result which is acquired by the management method and to which a value as a renewable product to a product selected depending on a content rate of renewable naphtha contained in a naphtha raw material is assigned is output.
[22] A computer-readable storage medium in which a computer program is stored, wherein
   a management program which allows a computer to implement the management method according to any of [13] to [19] is stored.
[23] A management program for allowing a computer to implement the management method according to any of [13] to [19].

### Advantageous Effect of Invention

According to the present invention, it is possible to provide a method capable of producing a monocyclic aromatic hydrocarbon such as benzene or para-xylene effective as a base raw material, with a naphtha raw material containing renewable naphtha.

According to the present invention, it is possible to provide a management method for a monocyclic aromatic hydrocarbon, usable in production of a monocyclic aromatic hydrocarbon with a naphtha raw material containing renewable naphtha, in which the method involves assigning a value as a renewable product such as renewable benzene or renewable para-xylene, to a monocyclic aromatic hydrocarbon product, depending on the content rate of the renewable naphtha contained in the naphtha raw material.

### BRIEF DESCRIPTION OF THE DRAWINGS

FIG. 1 is a schematic diagram for explaining the production method of the present invention.
FIG. 2 is a schematic diagram for explaining a process while step (A-1) is performed with a steam cracker (1).
FIG. 3 is a schematic diagram for explaining a process while step (A-3) is performed with a para-xylene production apparatus (4).
FIG. 4 is a schematic diagram for explaining the method for producing terephthalic acid of the present invention.
FIG. 5 is a schematic diagram for explaining the method for producing polyethylene terephthalate of the present invention.
FIG. 6 is a schematic diagram in which petroleum-derived light naphtha (also referred to as "conventional naphtha") and bio-naphtha are compared in terms of compositional ratio.
FIG. 7A is a schematic diagram for explaining step (V-1) in the management method of the present invention, in the case of the management method of the present invention, including step (V) and not including step (W).
FIG. 7B is a schematic diagram for explaining step (V-2) in the management method of the present invention, in the case of the management method of the present invention, including step (V) and not including step (W).
FIG. 7C is a schematic diagram for explaining step (V-3) in the management method of the present invention, in the case of the management method of the present invention, including step (V) and not including step (W).
FIG. 8A is a schematic diagram for explaining step (X-1) in the management method of the present invention, in the case of the management method of the present invention, including step (V) and not including step (W).
FIG. 8B is a schematic diagram for explaining step (X-2) in the management method of the present invention, in the case of the management method of the present invention, including step (V) and not including step (W).
FIG. 8C is a schematic diagram for explaining step (X-3) in the management method of the present invention, in the case of the management method of the present invention, including step (V) and not including step (W).
FIG. 8D is a schematic diagram for explaining step (X-4) in the management method of the present invention, in the case of the management method of the present invention, including step (V) and not including step (W).
FIG. 9A is a schematic diagram for explaining step (V-1) in the management method of the present invention, in the case of the management method of the present invention, including step (W) and not including step (V).
FIG. 9B is a schematic diagram for explaining step (V-2) in the management method of the present invention, in the case of the management method of the present invention, including step (W) and not including step (V).
FIG. 9C is a schematic diagram for explaining step (W-3) in the management method of the present invention, in the case of the management method of the present invention, including step (W) and not including step (V).
FIG. 9D is a schematic diagram for explaining step (W-4) in the management method of the present invention, in the case of the management method of the present invention, including step (W) and not including step (V).
FIG. 10A is a schematic diagram for explaining step (Y-1) in the management method of the present invention, in the case of the management method of the present invention, including step (W) and not including step (V).
FIG. 10B is a schematic diagram for explaining step (Y-2) in the management method of the present invention, in the case of the management method of the present invention, including step (W) and not including step (V).
FIG. 10C is a schematic diagram for explaining step (Y-3) in the management method of the present invention, in the case of the management method of the present invention, including step (W) and not including step (V).
FIG. 10D is a schematic diagram for explaining step (Y-4) in the management method of the present invention, in the case of the management method of the present invention, including step (W) and not including step (V).
FIG. 11A is a schematic diagram for explaining step (V-1) in the management method of the present invention, in the case of the management method of the present invention, including step (V) and step (W).
FIG. 11B is a schematic diagram for explaining step (V-2) in the management method of the present invention, in the case of the management method of the present invention, including step (V) and step (W).
FIG. 11C is a schematic diagram for explaining step (W-3) in the management method of the present invention, in the case of the management method of the present invention, including step (V) and step (W).
FIG. 11D is a schematic diagram for explaining step (V-3) in the management method of the present invention, in the case of the management method of the present invention, including step (V) and step (W).
FIG. 11E is a schematic diagram for explaining step (W-4) in the management method of the present invention, in the case of the management method of the present invention, including step (V) and step (W).
FIG. 12A is a schematic diagram for explaining step (Z-1) in the management method of the present invention, in the case of the management method of the present invention, including step (V) and step (W).
FIG. 12B is a schematic diagram for explaining step (Z-2) in the management method of the present invention, in the case of the management method of the present invention, including step (V) and step (W).
FIG. 12C is a schematic diagram for explaining step (Z-3) in the management method of the present invention, in the case of the management method of the present invention, including step (V) and step (W).
FIG. 12D is a schematic diagram for explaining step (Z-4) in the management method of the present invention, in the case of the management method of the present invention, including step (V) and step (W).
FIG. 12E is a schematic diagram for explaining another embodiment of step (Z) in the management method of the present invention, in the case of the management method of the present invention, including step (V) and step (W).
FIG. 13 is a schematic diagram for explaining a function configuration of a management apparatus.
FIG. 14 is a block diagram illustrating one example of a hardware configuration of a management apparatus.
FIG. 15 is a flowchart illustrating one example of a processing procedure of a management program in a control section of a management apparatus.
FIG. 16 is a flowchart illustrating a first variation of the processing procedure of a management program in a control section of a management apparatus.
FIG. 17 is a flowchart illustrating a second variation of the processing procedure of a management program in a control section of a management apparatus.

### DETAILED DESCRIPTION OF THE INVENTION

### (Definition)

Hereinafter, the present invention is described in more detail. The following terms are herein used.

The "renewable raw material" refers to a raw material from reproducible organic resources. The renewable raw material is not intended to be limited to only biological resource-derived products, and is widely interpreted as long as it falls into reproducible organic resources. For example, the renewable raw material is intended to also encompass even a petroleum-derived product which is derived from a recycled product with a petroleum-derived product as a raw material, such as a waste plastic-derived product.

The "renewable naphtha" refers to renewable raw material-derived naphtha.

The "renewable product" (renewable toluene, renewable para-xylene, or the like) refers to one to which a credit is assigned by a mass balance method.

The "biomass raw material" refers to a raw material from biological resources.

The "bio-naphtha" refers to biomass raw material-derived naphtha.

The "naphtha" refers to a group of hydrocarbons having 5 to 10 carbon atoms, obtained by refining petroleum.

The "xylene" is intended to encompass at least one of isomers (mixture) of para-xylene, ortho-xylene, or meta-xylene.

### (Method for producing monocyclic aromatic hydrocarbon)

The production method of the present invention is a method for producing at least one monocyclic aromatic hydrocarbon of benzene or xylene with a naphtha raw material containing renewable naphtha (hereinafter, sometimes simply referred to as "renewable naphtha raw material").

The renewable naphtha raw material includes at least renewable naphtha, and may further include non-renewable naphtha (conventional petroleum-derived naphtha).

Examples of the renewable naphtha include bio-naphtha made based on a biological raw material, and naphtha obtained by waste plastic treatment. In particular, bio-naphtha is more preferably used from the viewpoint of preservation of petroleum resources and reduction of carbon-dioxide emissions.

The bio-naphtha refers to, for example, naphtha made based on a biological raw material, for example, animal oil or vegetable oil, such as waste edible oil, beef tallow, palm oil, or tall oil (by-product of pulp and paper).

The renewable naphtha contained in the renewable naphtha raw material in the present invention is, in particular, preferably plant-derived bio-naphtha such as plant, wood, waste vegetable oil, or paper. Such bio-naphtha exhibits a compositional ratio close to that of a light fraction of petroleum-derived naphtha.

Specific examples of the monocyclic aromatic hydrocarbon to be produced in the production method of the present invention include benzene and xylene.

At least one monocyclic aromatic hydrocarbon of benzene or xylene produced by the production method of the present invention is produced with the renewable naphtha raw material, and thus is derived from the renewable naphtha.

For example, when a monocyclic aromatic hydrocarbon is produced with bio-naphtha as the renewable naphtha, according to the production method of the present invention with a bio-naphtha raw material containing the bio-naphtha, the monocyclic aromatic hydrocarbon produced contains a bio-naphtha-derived biomass component.

It can be confirmed by, for example, measurement of a radioactive carbon atom ¹⁴C that the monocyclic aromatic hydrocarbon contains a biomass component. In other words, xylene containing a radioactive carbon atom ¹⁴C can be obtained, for example, with bio-naphtha as a raw material.

Here, no differences in properties such as molecular weights, mechanical properties, and thermal properties are generated between biological compounds or compositions and petroleum-derived compounds or compositions. These compounds or compositions are distinguished generally with the degree of biomass. Since the petroleum-derived compound or composition contain no ¹⁴C (radioactive carbon 14, half-life 5730 years) in carbon atoms, the degree of biomass can be adopted and the concentration of ¹⁴C can be measured by accelerator mass spectrometry, to confirm whether a compound or composition produced is produced from only petroleum-derived naphtha or produced from a bio-naphtha-containing bio-naphtha raw material.

The degree of biomass is measured, for example, as follows. A sample to be measured is burned to generate carbon dioxide, and carbon dioxide purified in a vacuum line is reduced by hydrogen with iron as a catalyst, to produce graphite.

The graphite is applied to a ¹⁴C-AMS-dedicated apparatus (manufactured by NEC Corporation) based on a tandem accelerator, to count ¹⁴C and measure the concentration of ¹³C (¹³C/¹²C) and the concentration of ¹⁴C (¹⁴C/¹²C), and calculate the proportion of the concentration of ¹⁴C in carbon atoms in the sample with respect to standard modern carbon, from the measurement values. Here, oxalic acid (HOxII) provided from the National Institute of Standards and Technology (NIST) can be adopted as a standard sample.

The production method of the present invention includes at least
step (A-1) of pyrolyzing the naphtha raw material in the presence of water vapor, to produce and separate toluene, and
step (A-2) of subjecting toluene to disproportionation reaction or transalkylation reaction, to produce and separate at least one monocyclic aromatic hydrocarbon of benzene or xylene.

The production method of the present invention may further include
step (A-3) of subjecting xylene obtained in step (A-2), to adsorption separation or crystallization separation, to separate para-xylene.

The production method of the present invention may further include
a step of subjecting a residue containing at least one of ortho-xylene or meta-xylene after separation of para-xylene in step (A-3), to isomerization treatment, to produce para-xylene, and subsequently separating the para-xylene by adsorption separation or crystallization separation.

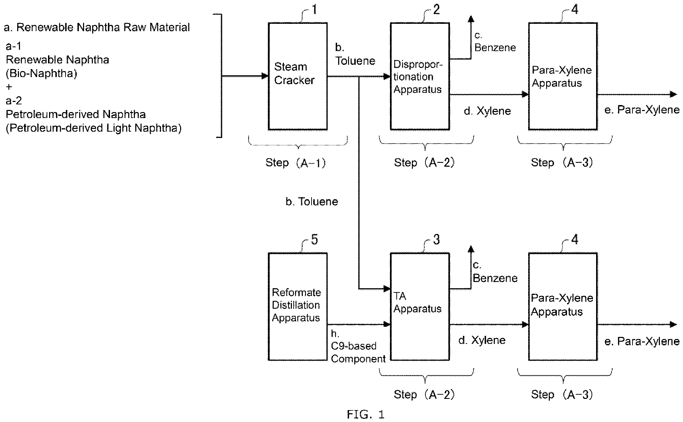

A preferred embodiment of the production method of the present invention is described with FIG. 1. In the drawing, an apparatus (1) which performs pyrolysis in the presence of water vapor is simply designated as "steam cracker (1)". An apparatus (2) which performs disproportionation reaction is designated as "disproportionation apparatus (2)", an apparatus (3) which performs transalkylation reaction is also designated as "TA apparatus (3)", and an apparatus (4) which performs adsorption separation or crystallization separation to separate para-xylene is also designated as "para-xylene production apparatus (4)". The disproportionation reaction means reaction for producing benzene C6 and xylene C8 from toluene C7, and the transalkylation reaction means reaction for producing benzene C6 and xylene C8 from toluene C7 and a C9-based component (monocyclic aromatic hydrocarbon having 9 carbon atoms) (h) and/or a C10-based component (monocyclic aromatic hydrocarbon having 10 carbon atoms) (i). The raw material in the transalkylation reaction may include, in addition to such C7-, C9-, and C10-based components, a component of C11 or more (monocyclic aromatic hydrocarbon having 11 or more carbon atoms).

In FIG. 1, a renewable naphtha raw material (a) containing renewable naphtha (for example, more specifically, bio-naphtha is used) (a-1) and petroleum-derived naphtha (for example, more specifically, petroleum-derived light naphtha is used) (a-2) is loaded to the steam cracker (1), to separate toluene (b) as a co-product of ethylene or the like, and the toluene (b) is loaded to the disproportionation apparatus (2) or the TA apparatus (3), to separate benzene (c). Thus, benzene (c) derived from the renewable naphtha raw material (a) can be produced.

In this regard, the toluene (b) can be loaded to the disproportionation apparatus (2) or loaded to the TA apparatus (3) taken together with a C9-based component (h), to separate xylene (d), thereby producing xylene (d) derived from the renewable naphtha raw material (a).

The xylene (d) can be loaded to the para-xylene production apparatus (4), to separate para-xylene (e), thereby producing para-xylene (e) derived from the renewable naphtha raw material (a).

A chemical industrial process conventionally known does not produce any monocyclic aromatic hydrocarbon by combining such steam cracker (1), and disproportionation apparatus (2) or TA apparatus (3), and a sequential process of naphtha -> steam cracker (1) -> toluene -> disproportionation apparatus (2) -> benzene or xylene -> para-xylene, or naphtha -> steam cracker (1) -> toluene -> TA apparatus (3) -> benzene or xylene -> para-xylene is a new process not usually performed. This new process can produce a monocyclic aromatic hydrocarbon derived from the renewable naphtha raw material (a).

The production method of the present invention, illustrated in FIG. 1, is described below in more detail.

A method for producing at least one monocyclic aromatic hydrocarbon of benzene or xylene with a renewable naphtha raw material includes the following step (A-1) and the following step (A-2).
Step (A-1) is a step of loading a renewable naphtha raw material (a) to a steam cracker (1), to produce and separate toluene (b), and
step (A-2) is a step of loading the toluene (b) to a disproportionation apparatus (2), or to a TA apparatus (3), together with a C9-based component (h), to produce and separate benzene (c) and xylene (d).

The production method further includes the following step (A-3) of producing para-xylene.

Step (A-3) is a step of loading the xylene (d) to a para-xylene production apparatus (4), to separate para-xylene (e).

An apparatus used in the production method of the present invention is described in further detail.

### <Steam cracker (1)>

The raw material to be loaded to the steam cracker (1) is a renewable naphtha raw material (a). A preferred aspect of the renewable naphtha raw material (a) is a renewable naphtha raw material (a) containing renewable naphtha (a-1) and petroleum-derived light naphtha (a-2), more preferably a bio-naphtha raw material (a) containing bio-naphtha (a-1) and petroleum-derived light naphtha (a-2).

The content rate of the renewable naphtha (a-1) contained in the renewable naphtha raw material (a) is not particularly restricted, and can be appropriately selected depending on the object.

The raw material to be loaded to the steam cracker (1) can be light oil, besides the renewable naphtha raw material (a). The light oil here used is preferably biomass raw material-derived light oil such as biodiesel.

Examples of the biomass raw material include vegetable oils such as canola oil, palm oil, olive oil, sunflower oil, soybean oil, and rice oil, animal oils and fats, such as beef tallow, lard, and fish oil, and waste edible oil.

The mixing proportion of the renewable naphtha raw material (a) and the biomass raw material-derived light oil in the raw material to be loaded to the steam cracker (1) is not particularly restricted, and can be appropriately selected depending on the object.

The steam cracker (1) is an apparatus capable of pyrolyzing the renewable naphtha raw material (a) in the presence of water vapor and thus producing and separating toluene being a co-product of ethylene or the like.

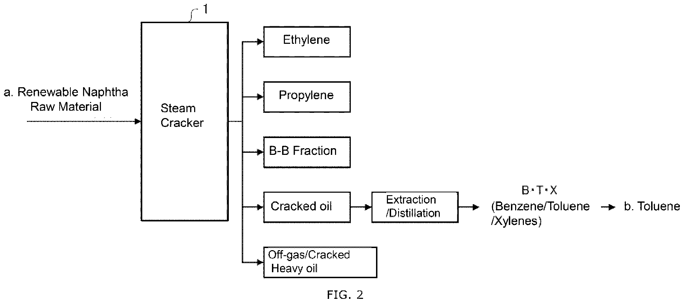

A more detailed process in step (A-1) performed with the steam cracker (1) is described with FIG. 2.

A component having an aromatic compound, in a product obtained by loading the renewable naphtha raw material (a) to the steam cracker (1), is subjected to distillation or extraction, and purification by separation, thereby obtaining renewable component-containing toluene (b).

The cracking temperature in the steam cracker is preferably 750°C to 900°C, more preferably 770°C to 850°C.

In order to efficiently perform running of the steam cracker, the retention time (reaction time) of the raw material is preferably 0.1 to 0.5 seconds, more preferably 0.1 to 0.3 seconds. The steam/raw material (mass ratio) is preferably 0.2 to 0.9, more preferably 0.3 to 0,7.

### < Disproportionation apparatus (2)>

In a disproportionation apparatus (2), reaction in which methyl groups are transferred between toluene and toluene results in conversion of toluene (b) to benzene and xylene, and production and separation of benzene (c) and xylene (d) (step (A-2)). The toluene (b) may be toluene obtained in the steam cracker (1), may be other toluene (for example, petroleum-derived toluene), or may be a mixture of toluene obtained in the steam cracker (1) and other toluene.

### <TA apparatus (3)>

In a TA apparatus (3), reaction in which methyl groups are transferred between the toluene (b) and a C9-based component and/or a C10-based component results in conversion of the toluene (b), a C9-based component (h) and a C10-based component (i) to benzene and xylene and thus production and separation of benzene (c) and xylene (d) (step (A-2)). The toluene (b) may be toluene obtained in the steam cracker (1), may be other toluene (for example, petroleum-derived toluene), or may be a mixture of toluene obtained in the steam cracker (1) and such other toluene.

The raw material in the transalkylation reaction may include, in addition to such toluene (b), C9-based component (h), and C10-based component (i), a component of C11 or more.

Examples of the C9-based component include trimethylbenzene and methylethylbenzene, examples of the C10-based component include tetramethylbenzene and ethylxylene, and examples of the component of C11 or more include trimethylethylbenzene and diethyltoluene.

### <Para-xylene production apparatus (4)>

In a para-xylene production apparatus (4), the xylene (d) (more specifically, mixed xylene including meta-xylene, ortho-xylene, para-xylene, and the like) obtained in the disproportionation apparatus (2) or the TA apparatus (3) is subjected to adsorption separation or crystallization separation, to separate para-xylene (step (A-3)).

Step (A-3) can further include a step of subjecting a residue containing at least one of ortho-xylene or meta-xylene after separation of para-xylene, to isomerization treatment, to produce para-xylene, and subsequently performing adsorption separation or crystallization separation again, to separate para-xylene.

### (Production of para-xylene by adsorption separation method)

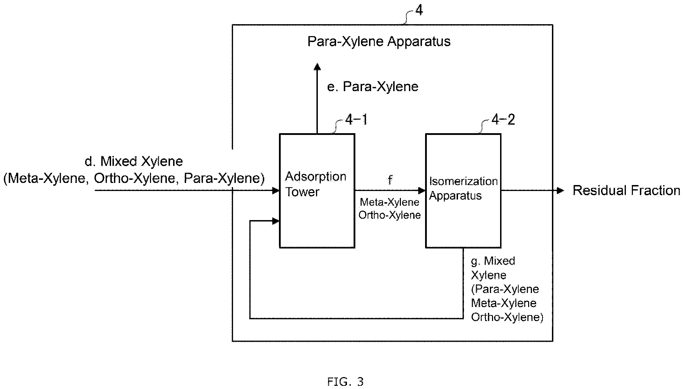

A preferred embodiment of the para-xylene production apparatus (4) is described with FIG. 3.

In an adsorption separation method, the xylene (d) (more specifically, mixed xylene in which meta-xylene, ortho-xylene, para-xylene, and the like are mixed) is loaded to an adsorption tower (4-1) in the para-xylene production apparatus (4) in which para-xylene is adsorbed and separated with an adsorbent. Para-xylene (e) is separated in the adsorption tower (4-1).

A residue (f) containing ortho-xylene and meta-xylene, remaining after para-xylene separation, is loaded to an isomerization apparatus (4-2) in the para-xylene production apparatus (4), isomerization treatment is performed, and para-xylene-containing mixed xylene (g) (more specifically, mixed xylene after isomerization treatment, in which meta-xylene, ortho-xylene, para-xylene, and the like are mixed) obtained after isomerization treatment is again subjected to the adsorption tower (4-1). Para-xylene (e) is separated in the adsorption tower (4-1).

This step of separating the para-xylene (e) through isomerization apparatus -> adsorption tower can be repeatedly performed. Once an objective amount of the para-xylene (e) can be separated, such a repeated operation is terminated.

### (Production of para-xylene by crystallization separation method)

In a crystallization separation method, para-xylene and meta-xylene/ortho-xylene are separated from the xylene (d) (more specifically, mixed xylene in which meta-xylene, ortho-xylene, para-xylene, and the like are mixed), by use of the difference in melting point. The mixed xylene to be loaded to the para-xylene production apparatus is cooled to crystallize para-xylene having a higher melting point than other xylene isomers and the crystallized product is subjected to centrifugation, crystal cleaning, and recrystallization steps, and thus para-xylene is separated.

### (Method for producing terephthalic acid)

The production method of the present invention is a method for producing terephthalic acid with a renewable naphtha raw material and/or biomass raw material-derived light oil, and includes step (A-3) of obtaining para-xylene, and step (A-4) of oxidizing the para-xylene, to obtain terephthalic acid. Step (A-3), in which the renewable naphtha raw material and para-xylene are obtained, is as described above. The renewable naphtha raw material here used is preferably bio-naphtha, and the biomass raw material-derived light oil here used is preferably biodiesel.

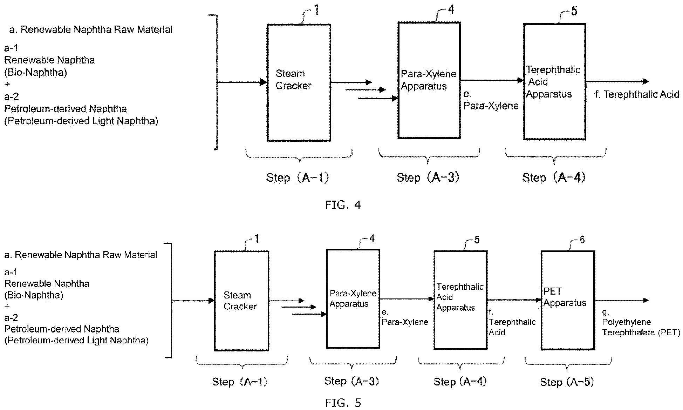

A preferred aspect of the present invention is described with reference to FIG. 4. As illustrated in FIG. 4, the renewable naphtha raw material (a) containing bio-naphtha is loaded to the steam cracker (1) and undergoes each reaction described above, and thereafter para-xylene is obtained from the para-xylene apparatus (4). Subsequently, the para-xylene obtained is loaded to a terephthalic acid production apparatus (5), to obtain terephthalic acid (step (A-4)).

According to the production method of the present invention, it is possible to use bio-naphtha and/or biomass raw material-derived light oil in the renewable naphtha raw material and thus obtain terephthalic acid containing radioactive carbon atom ¹⁴C.

### <Terephthalic acid production apparatus (5)>

In a terephthalic acid production apparatus (5), the para-xylene obtained in the para-xylene production apparatus (4) is oxidized, to produce terephthalic acid (step (A-4)).

In step (A-4), a conventionally known method is used to oxidize para-xylene, to produce terephthalic acid. For example, para-xylene is subjected to liquid-phase oxidation by molecular oxygen in an acetic acid solvent in the presence of a catalyst, thereby producing terephthalic acid. The catalyst here used is a catalyst conventionally known to be used in the oxidation reaction, and specific examples thereof include heavy metal compounds such as a cobalt compound, a manganese compound, an iron compound and a chromium compound, and a bromine compound. The catalyst, when is in a molten or dissolved state during the oxidation reaction, is higher in reaction rate, and thus is preferably in a molten or dissolved state in the reaction system. Conditions in the oxidation reaction can be appropriately set.

### (Method for producing polyethylene terephthalate)

The production method of the present invention is a method for producing polyethylene terephthalate with a renewable naphtha raw material and/or biomass raw material-derived light oil, and includes step (A-4) of obtaining terephthalic acid, and step (A-5) of obtaining polyethylene terephthalate by condensation reaction of terephthalic acid and ethylene glycol. The renewable naphtha raw material, and step (A-4) of obtaining terephthalic acid are as described above. The renewable naphtha raw material here used is preferably bio-naphtha, and the biomass raw material-derived light oil here used is preferably biodiesel.

A preferred aspect of the present invention is described with reference to FIG. 5. As illustrated in FIG. 5, the renewable naphtha raw material (a) containing bio-naphtha is loaded to the steam cracker (1) and undergoes each reaction described above, and thereafter terephthalic acid is obtained from the terephthalic acid production apparatus (5) (step (A-4)). Subsequently, the terephthalic acid obtained is loaded to a polyethylene terephthalate (PET) production apparatus (6), to obtain polyethylene terephthalate (PET) (step (A-5)).

According to the production method of the present invention, it is possible to use bio-naphtha and/or biomass raw material-derived light oil in the renewable naphtha raw material and thus obtain polyethylene terephthalate containing radioactive carbon atom ¹⁴C.

The polyethylene terephthalate obtained can be used in a conventionally known application, and can be utilized in, for example, a preform, a PET bottle, a shrink film, and a stretch film.

### <Polyethylene terephthalate (PET) production apparatus (6)>

In a polyethylene terephthalate production apparatus (6), polyethylene terephthalate is produced by condensation reaction of the terephthalic acid obtained in the terephthalic acid production apparatus (5), and ethylene glycol (step (A-5)).

In step (A-5), a conventionally known method is used to produce polyethylene terephthalate by condensation reaction of the terephthalic acid and ethylene glycol. For example, polyethylene terephthalate can be produced by condensation reaction of the terephthalic acid and ethylene glycol (dehydration-condensation) in the presence of a polymerization catalyst. Examples of the polymerization catalyst preferably include respective metal compounds containing titanium, zirconium, germanium, zinc, aluminum, magnesium and calcium, and any mixture thereof, particularly preferably include a titanium compound, a zirconium compound and a germanium compound. The polymerization catalyst, when in a molten or dissolved state during the condensation reaction, is higher in reaction rate, and thus is preferably a liquid or a compound to be dissolved in an ester low polymer or polyester during the condensation reaction. Conditions in the condensation reaction can be appropriately set.

Ethylene glycol, which is one raw material in polyethylene terephthalate (PET) production in step (A-5) may be derived from petroleum, may be derived from a biomass raw material, or may be bio-ethylene glycol utilizing a mass balance system. Such biomass raw material-derived ethylene glycol can be used in step (A-5), thereby resulting in an enhancement in degree of biomass of the resulting polyethylene terephthalate.

### <Features of production method of the present invention>

According to the production method of the present invention, a petroleum-derived raw material can be partially or fully replaced with a renewable raw material such as a biomass raw material or a recycled raw material, thereby not only allowing for preservation of petroleum resources, and reduction of environmental burden such as carbon-dioxide emissions, but also providing a monocyclic aromatic hydrocarbon product produced, which has a compositional ratio comparable with that of a conventional monocyclic aromatic hydrocarbon product produced with a petroleum-derived raw material.

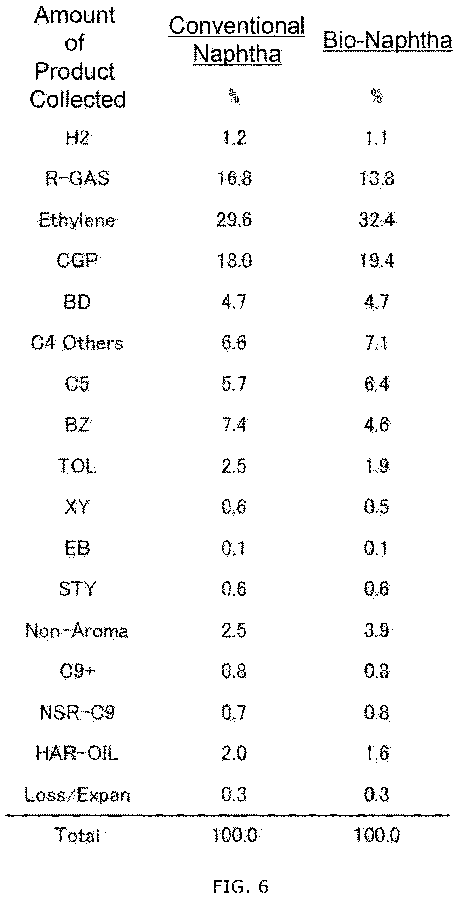

More specifically, the exemplified compositional ratios of petroleum-derived light naphtha (also referred to as "conventional naphtha") and bio-naphtha are approximated as illustrated in FIG. 6. In the production method of the present invention, bio-naphtha whose compositional ratio is close to that of conventional naphtha can be used and raw material naphtha in which the conventional naphtha is partially or fully replaced with bio-naphtha can be used, thereby allowing for production of a monocyclic aromatic hydrocarbon whose compositional ratio is comparable with that of a product from the conventional naphtha in which the yield of a product from the bio-naphtha is not different from, but comparable with the yield of a product from the conventional naphtha. The quality of the monocyclic aromatic hydrocarbon produced can also not be different from, but be comparable with the quality of a monocyclic aromatic hydrocarbon produced from the conventional naphtha.

While not only the compositional ratio of a final product of the monocyclic aromatic hydrocarbon in the production method of the present invention is confirmed, but also the compositional ratio of each co-product including toluene obtained from the steam cracker is confirmed, there is no difference in compositional ratio of each co-product between a case of conventional naphtha containing bio-naphtha and a case of only conventional naphtha.

### (Method for managing renewable monocyclic aromatic hydrocarbon)

In recent years, an approach "mass balance system" (hereinafter, also referred to as "material balance system") has attracted attention in the chemical industry in order to sell an environment-conscious product with a plant-derived raw material or a recycled material.

It is desired to perform the assignment of a value as a renewable product, with a mass balance system, also to a monocyclic aromatic hydrocarbon produced with the method for producing a renewable component-containing monocyclic aromatic hydrocarbon of the present invention, by a simple and reliable method.

The present invention then provides a management method which, when a monocyclic aromatic hydrocarbon is produced with the method for producing a renewable component-containing monocyclic aromatic hydrocarbon of the present invention, can assign a value as a renewable product, to such a monocyclic aromatic hydrocarbon product, depending on the content rate of the renewable naphtha contained in the renewable naphtha raw material, by a simple and reliable method.

The mass balance system here refers to, for example, a procedure in which, when a raw material having specific properties, such as a biomass raw material, is mixed with a raw material not having such properties in a distribution/processing step from a raw material to a products, such properties are assigned as a credit, to a portion of the products, depending on the content rate of the raw material having such properties.

For example, in a case where benzene is produced by loading a renewable naphtha (bio-naphtha) raw material to a steam cracker, such benzene contains a renewable component (biomass component), but a biomass fraction in the renewable naphtha raw material loaded to the steam cracker is simultaneously distributed to each co-product (co-product, no renewable component can be allocated to a specific product). The mass balance system is here adopted in order to virtually assign a biomass fraction as a credit, to a portion of the products such as benzene and para-xylene. A specific method for such an assignment is described below.

The mass balance system (material balance system) is a system for arbitrary assignment with a biomass fraction as a credit by a manufacturer, and thus the legitimacy is generally verified with the authentication by a third-party certifier. The third-party certifier is, for example, ISCC (International Sustainability & Carbon Certification) or RSB (Principles & Criteria for Sustainable Biofuel Production).

The method for managing a renewable monocyclic aromatic hydrocarbon of the present invention can be used in production of at least one monocyclic aromatic hydrocarbon of benzene or xylene with a renewable naphtha-containing renewable naphtha raw material.

The management method of the present invention is a method for assigning a value as a renewable product, to at least one monocyclic aromatic hydrocarbon product, depending on the content rate of the renewable naphtha contained in the renewable naphtha raw material.

The management method of the present invention includes at least one or more steps selected from step (V) of confirming that xylene and/or benzene are/is produced and step (W) of confirming that para-xylene is obtained.

Hereinafter, the management method including step (V) and/or step (W) is described with respect to each case in detail.

### <Management method including step (V) and not including step (W) >

Step (V) of confirming that xylene and/or benzene are/is produced includes the following step (V-1), the following step (V-2), and the following step (V-3).

In a case where the management method of the present invention includes step (V) and does not include step (W), the management method includes step (X) of confirming the proportion of a product to which a value as a renewable product is to be assigned.

Step (X) includes the following step (X-1), the following step (X-2), the following step (X-3), and the following step (X-4).

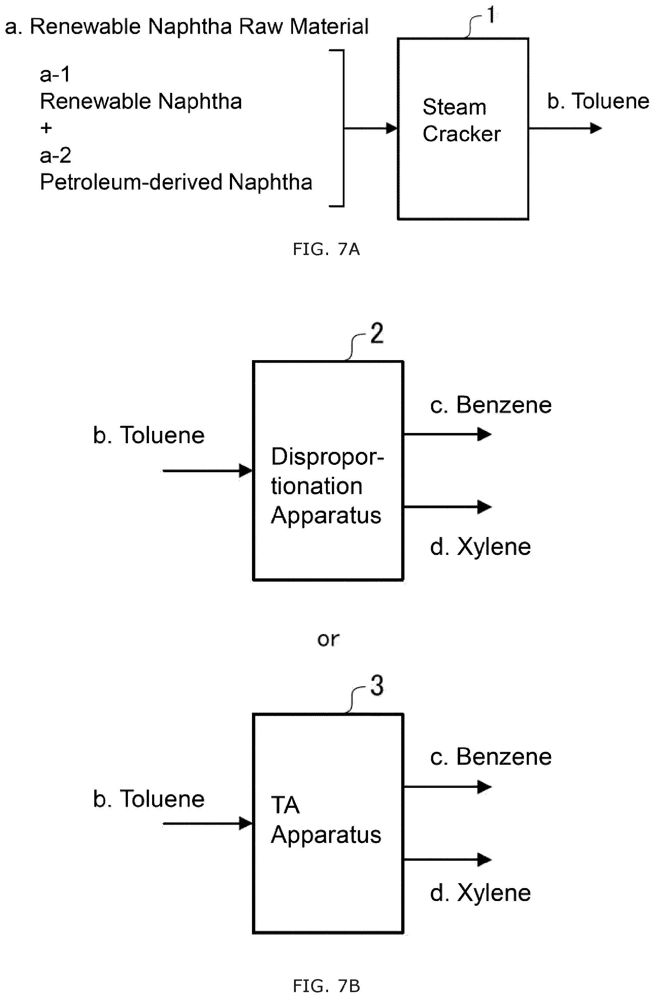

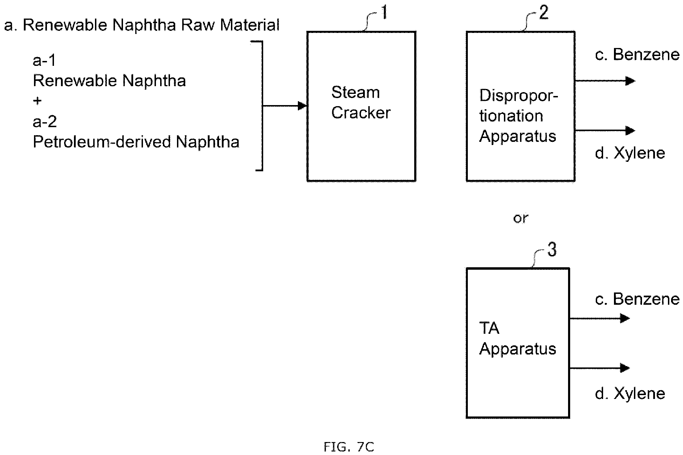

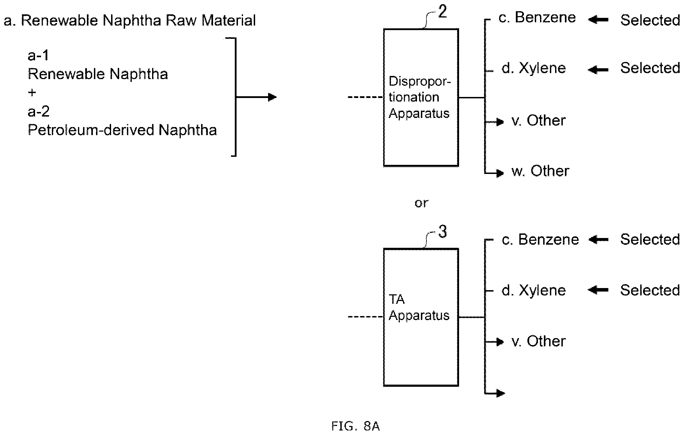

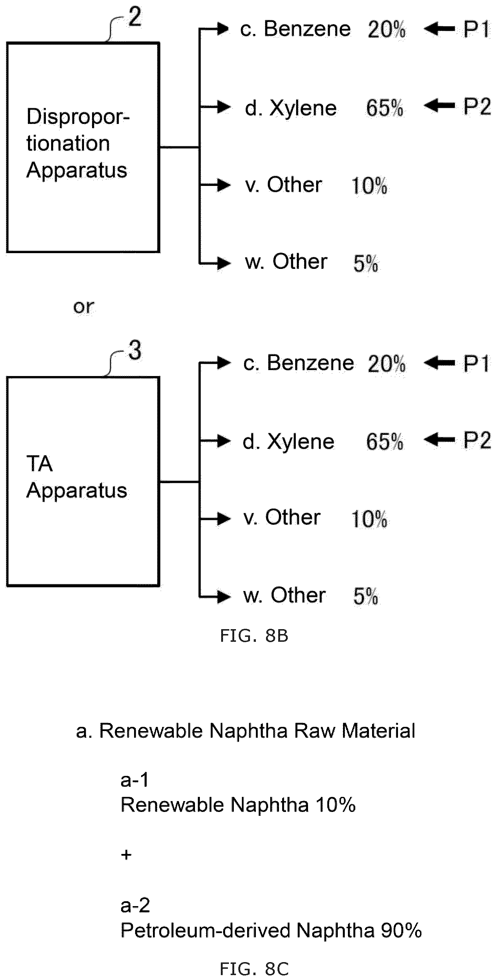

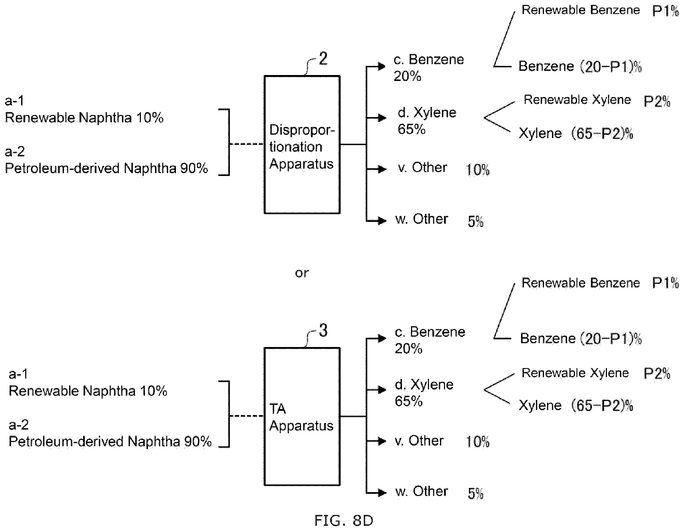

Preferred embodiments of the management method of the present invention, the method including step (V) and not including step (W), are described with FIG. 7A to FIG. 7C (these are also collectively referred to as "FIG. 7") and FIG. 8A to FIG. 8D (these are also collectively referred to as "FIG. 8").

Step (V-1) is a step of confirming that toluene (b) is produced from the renewable naphtha raw material (a) loaded to the steam cracker (1), as illustrated in FIG. 7A. In other words, it is confirmed whether or not a desired output (toluene) is achieved from an input (renewable naphtha raw material) in the steam cracker.

Step (V-2) is a step of confirming that xylene (d) and/or benzene (c) are/is produced from toluene (b) loaded to the disproportionation apparatus (2), or taken together with the C9-based component (h) and/or the C10-based component and then loaded to the TA apparatus (3), as illustrated in FIG. 7B. In other words, it is confirmed whether or not a desired output (xylene and/or benzene) is achieved from an input (toluene (optionally including confirmation of an input of the C9-based component and/or the C10-based component)) in the disproportionation apparatus (2) or the TA apparatus (3). The toluene (b) loaded to the disproportionation apparatus (2) or the TA apparatus (3) in step (V-2) may be the toluene (b) obtained in step (V-1), may be other toluene (for example, petroleum-derived toluene), or may be a mixture of the toluene (b) obtained in step (V-1), and other toluene, and is preferably other toluene.

Step (V-3) is a step of confirming that xylene (d) and/or benzene (c) are/is produced from the renewable naphtha raw material (a) by treatment in the steam cracker (1), the disproportionation apparatus (2) or the TA apparatus (3) in the listed order, as illustrated in FIG. 7C. In other words, it is confirmed whether or not a desired output (xylene and/or benzene) is achieved from an input (renewable naphtha raw material) in the case of reaction in the steam cracker (1), the disproportionation apparatus (2) or the TA apparatus (3) in the listed order. The toluene (b) loaded to the disproportionation apparatus (2) or the TA apparatus (3) in step (V-3) is not illustrated, but is the same as the toluene (b) in step (V-2).

Step (X-1) is a step of selecting one or more products to be assigned to renewable product(s), in products produced by the disproportionation apparatus (2) or the TA apparatus (3).

Step (X-2) is a step of determining the value of the proportion (P) of the product(s) to be assigned to renewable product(s) in the proportion of the products selected in step (X-1), with respect to the products obtained by the disproportionation apparatus (2) or the TA apparatus (3).

Step (X-3) is a step of grasping the value of the content rate (Q) of the renewable naphtha contained in the renewable naphtha raw material.

Step (X-4) is a step of comparing the value of the proportion (P) and the value of the content rate (Q) and confirming that the value of the proportion (P) is equal to or less than the value of the content rate (Q).

Step (X-1) to step (X-4) (these steps are also collectively referred to as "step (X)") are specifically described with reference to FIG. 8.

Step (X-1) is to select one or more desired products to be assigned to renewable product(s), in products (for example, benzene (c), xylene (d), and other product(s) (for example, (v) and/or (w))) produced by the apparatus to be finally used (here, the disproportionation apparatus (2) or the TA apparatus (3)) in the reaction step of providing desired products, as illustrated in FIG. 8A. For example, one benzene may be selected, one xylene may be selected, or two, benzene and xylene, may be selected.

Step (X-2) is to determine the value of the proportion (P) to be assigned as the renewable product(s), to the desired product(s) selected in step (X-1), in products produced from the disproportionation apparatus (2) or the TA apparatus (3), as illustrated in FIG. 8B. For example, it is assumed as illustrated in FIG. 8B that the products produced from the disproportionation apparatus (2) are benzene (c), xylene (d) and other product(s) ((v) and/or (w)) and the respective proportions of the products satisfy benzene (c): 20% by mass, xylene (d): 65% by mass, other product (v): 10% by mass and other product (w): 5% by mass. There is described for convenience under the assumption that also the products produced from the TA apparatus (3) are those produced at the same product proportions as in the disproportionation apparatus (2). In other words, it is assumed as illustrated in FIG. 8B that the products produced from the TA apparatus (3) are benzene (c), xylene (d) and other product(s) ((v) and/or (w)) and the respective proportions of the products satisfy benzene (c): 20% by mass, xylene (d): 65% by mass, other product (v): 10% by mass and other product (w): 5% by mass.

Here, in a case where benzene is selected in step (X-1), the proportion (P1) in 20% by mass of the benzene product, to be assigned to renewable benzene, is determined. Alternatively, in a case where xylene is selected in step (X-1), the proportion (P2) in 65% by mass of the xylene product, to be assigned to renewable xylene, is determined. Alternatively, in a case where two, benzene and xylene, are selected in step (X-1), the proportion (P1) in 20% by mass of the benzene product, to be assigned to renewable benzene, and the proportion (P2) in 65% by mass of the xylene product, to be assigned to renewable xylene, are determined.

Step (X-3) is to grasp the value of the content rate (Q) of the renewable naphtha (a-1) contained in the renewable naphtha raw material (a), as illustrated in FIG. 8C. For example, as illustrated in FIG. 8C, in a case where the renewable naphtha raw material (a) contains 10% by mass of the renewable naphtha (a-1) and 90% by mass of the petroleum-derived naphtha (a-2), the content rate of the renewable naphtha (a-1) is grasped to be 10% by mass.

Step (X-4) is to compare the value of the proportion (P) and the value of the content rate (Q) and confirm that the value of the proportion (P) is equal to or less than the value of the content rate (Q), and when the content rate (Q) of the renewable naphtha (a-1) is assumed to be 10% by mass as illustrated in FIG. 8D, the proportion (P), to be assigned to the renewable product(s), to the product(s) is 10% by mass or less.

In a case where two or more products to be assigned to renewable products are selected, the proportion (P) to be assigned means the total value of the proportions to be respectively assigned to the products selected.

In a case where only benzene is selected in step (X-1), the proportion (P1) to be assigned to renewable benzene is the proportion (P), in a case where only xylene is selected in step (X-1), the proportion (P2) to be assigned to renewable xylene is the proportion (P), and in a case where two, benzene and xylene, are selected in step (X-1), the total of the proportion (P1) to be assigned to renewable benzene and the proportion (P2) to be assigned to renewable xylene is the proportion (P).

In other words, under the assumption that the content rate (Q) of the renewable naphtha (a-1) is 10% by mass, 10% by mass of benzene in 20% by mass of the benzene product can be assigned to renewable benzene in a case where benzene is selected as a product to be assigned to a renewable product in step (X-1). Whether or not the proportion (P1) to be assigned to renewable benzene exceeds the content rate (Q) of the renewable naphtha (a-1) (10% by mass in an example of FIG. 8D) is confirmed in step (X-4).

In a case where xylene is selected as a product to be assigned to a renewable product in step (X-1), 10% by mass of xylene in 65% by mass of the xylene product can be assigned to renewable xylene. Whether or not the proportion (P2) to be assigned to renewable xylene exceeds the content rate (Q) of the renewable naphtha (a-1) (10% by mass in an example of FIG. 8D) is confirmed in step (X-4).

In a case where two, benzene and xylene, are selected as products to be assigned to renewable products in step (X-1), (P1) (% by mass) in 20% by mass of the benzene product and (P2) (% by mass) in 65% by mass of the xylene product can be respectively assigned to renewable benzene and renewable xylene. For example, In a case where P1 is 5% by mass and P2 is 5% by mass, the total is 10% by mass or less and thus such proportions can be assigned. Alternatively, for example, in a case where P1 is 1% by mass and P2 is 9% by mass, the total is 10% by mass or less and thus such proportions can be assigned. On the other hand, for example, in a case where P1 is 6% by mass and P2 is 7% by mass, the total is 13% by mass and exceeds 10% by mass, and thus such proportions cannot be assigned. Whether or not the total of the proportion (P1) to be assigned to renewable benzene and the proportion (P2) to be assigned to renewable xylene exceeds the content rate (Q) of the renewable naphtha (a-1) (10% by mass in an example of FIG. 8D) is confirmed in step (X-4).

It is thus possible to assign a value as a renewable product, to the desired product(s) selected in step (X-1), in products produced from the disproportionation apparatus (2) or the TA apparatus (3), depending on the content rate of the renewable naphtha contained in the renewable naphtha raw material.

Each product produced by the production method of the invention of the present application contains a renewable component depending on the content rate of the renewable naphtha contained in the renewable naphtha raw material, and a renewable component is distributed to such each product. In other words, the content of such a renewable component is according to the yield in production from each apparatus. The mass balance system can be utilized to handle a specific product as a renewable product to which 100% of such a renewable component is assigned, within the content rate of renewable naphtha contained in the renewable naphtha raw material.

In other words, with FIG. 8D as an example, it can be determined that (P1) (% by mass) of the benzene product in 20% by mass of the benzene product corresponds to renewable benzene whose 100% corresponds to a renewable component and (P2) (% by mass) of the xylene product in 65% by mass of the xylene product corresponds to renewable xylene whose 100% corresponds to a renewable component. The total of (P1) (% by mass) and (P2) (% by mass) is here required not to exceed 10% by mass in FIG. 8D.

According to the mass balance system, in FIG. 8D, a benzene product (20 - (P1) (% by mass) of benzene product) in which (P1) (% by mass) is subtracted from 20% by mass of the benzene product also actually contains a renewable component. However, the benzene product is determined not to be handled as renewable benzene because a credit of 100% renewable benzene is assigned to (P1) (% by mass) of benzene. A xylene product (65 - (P2) (% by mass) of xylene product) in which (P2) (% by mass) is subtracted from 65% by mass also actually contains a renewable component again, but the xylene product is determined not to be handled as renewable xylene because a credit of 100% renewable xylene is assigned to (P2) (% by mass) of xylene. The assignment is made by auditing the administrative operation system of a manufacturer, and issuing the authentication, if a predetermined standard is satisfied, to secure the correctness, by a third-party certifier such as ISCC (International Sustainability & Carbon Certification) or RSB (Principles & Criteria for Sustainable Biofuel Production).

According to the management method of the present invention, it is possible to simply and reliably perform the assignment of a value as a renewable product, with a mass balance system, also to a monocyclic aromatic hydrocarbon produced with the method for producing a renewable component-containing monocyclic aromatic hydrocarbon of the present invention, and secure the correctness of the assignment result of the value by a third-party certifier.

A value of 10% by mass as the content rate (Q) of the renewable naphtha (a-1), and a value of 20% by mass of the benzene product and a value of 65% by mass of the xylene product, described in FIG. 8, are values set for convenience in order to facilitate understanding of the present invention, and are not limited to these values. The content rate (Q) of the renewable naphtha (a-1) may be, for example, 1% by mass or 30% by mass, and a desired content rate (Q) may be set depending on the object. The proportion of production of each product also varies depending on reaction conditions or the like, and therefore reaction conditions or the like may be appropriately adjusted so that each product is produced at a desired proportion of production.

Even in a case where the management method of the present invention
- includes step (W) of confirming that para-xylene is obtained and does not include step (V), or
- includes both step (V) and step (W),
the same management method as described in the case where step (V) is included and step (W) is not included can be applied.

Hereinafter, there are also described the management method including step (W) and not including step (V) and the management method including step (V) and step (W).

### <Management method including step (W) and not including step (V) >

Step (W) of confirming that para-xylene is obtained includes the following step (V-1), the following step (V-2), the following step (W-3), and the following step (W-4). Step (V-1) and step (V-2) are as described in the section <Management method including step (V) and not including step (W)>.

In a case where the management method of the present invention includes step (W) and does not include step (V), the management method includes step (Y) of confirming a proportion of a product to which a value as a renewable product is to be assigned.

Step (Y) includes the following step (Y-1), the following step (Y-2), the following step (Y-3), and the following step (Y-4).

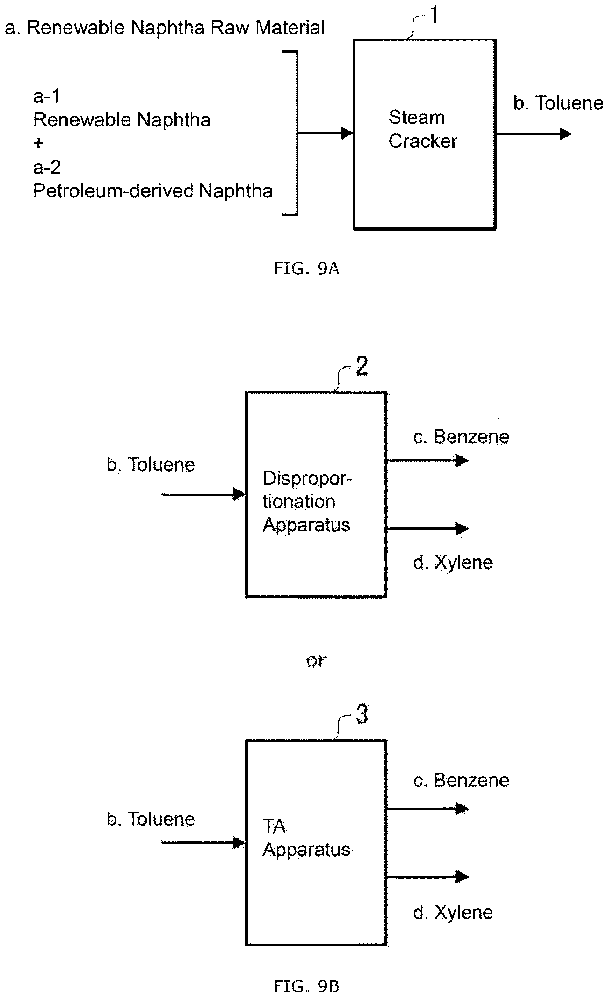

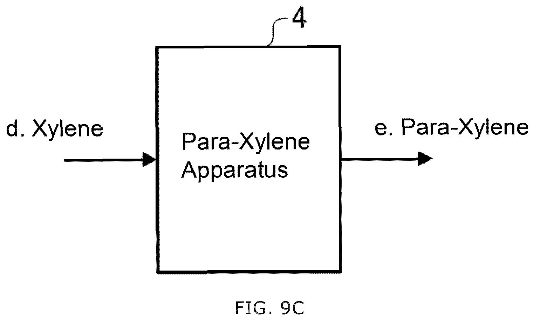

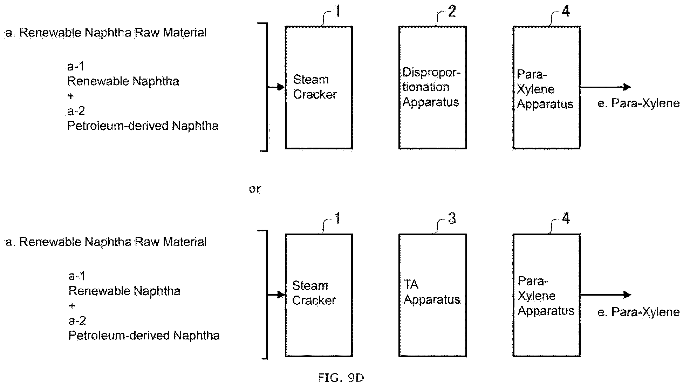

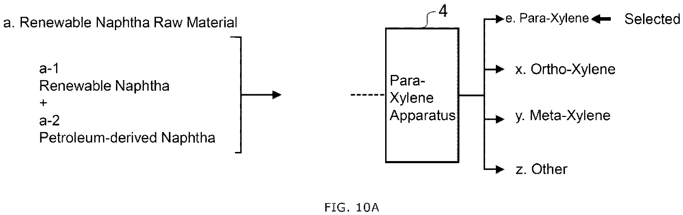

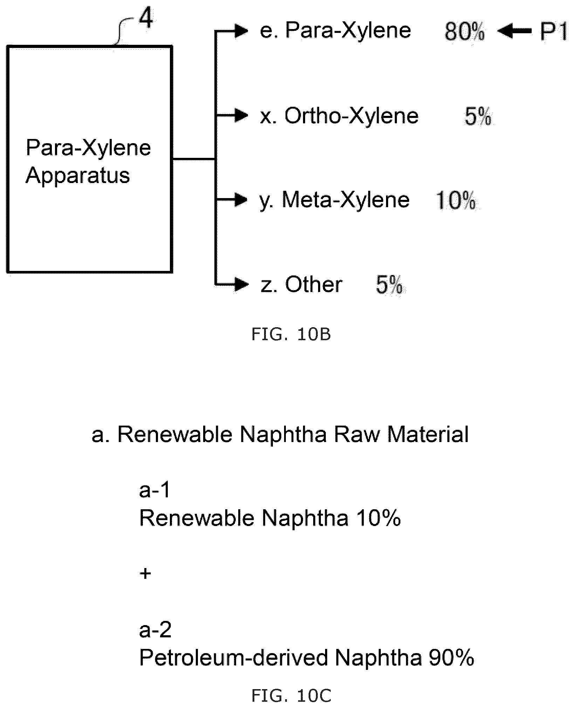

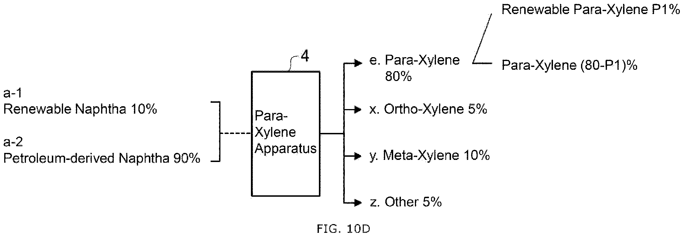

Preferred embodiments of the management method of the present invention, the method including step (W) and not including step (V), are described with FIG. 9A to FIG. 9C (these are also collectively referred to as "FIG. 9") and FIG. 10A to FIG. 10D (these are also collectively referred to as "FIG. 10").

Step (V-1) is a step of confirming that toluene (b) is produced from the renewable naphtha raw material (a) loaded to the apparatus (1) for pyrolysis in the presence of water vapor (steam cracker), as illustrated in FIG. 9A. In other words, it is confirmed whether or not a desired output (toluene) is achieved from an input (renewable naphtha raw material) in the apparatus for pyrolysis in the presence of water vapor (steam cracker).

Step (V-2) is a step of confirming that xylene (d) and/or benzene (c) are/is produced from toluene (b) loaded to the disproportionation apparatus (2), or taken together with the C9-based component (h)) and then loaded to the TA apparatus (3), as illustrated in FIG. 9B. In other words, it is confirmed whether or not a desired output (xylene and/or benzene) is achieved from an input (toluene (optionally including confirmation of an input of the C9-based component and/or the C10-based component)) in the disproportionation apparatus (2) or the TA apparatus (3). The toluene (b) loaded to the disproportionation apparatus (2) or the TA apparatus (3) in step (V-2) may be the toluene (b) obtained in step (V-1), may be other toluene (for example, petroleum-derived toluene), or may be a mixture of the toluene (b) obtained in step (V-1), and other toluene, and is preferably other toluene.

Step (W-3) is a step of confirming that para-xylene (e) is obtained from xylene (d) loaded to the para-xylene production apparatus (4), as illustrated in FIG. 9C. In other words, it is confirmed whether or not a desired output (para-xylene) is achieved from an input (xylene) in the para-xylene production apparatus (4).

Step (W-4) is a step of confirming that para-xylene (e) is obtained from the renewable naphtha raw material (a) by treatment in the apparatus for pyrolysis in the presence of water vapor (steam cracker) (1), the disproportionation apparatus (2) or the TA apparatus (3), and the para-xylene production apparatus (4) in the listed order, as illustrated in FIG. 9D. In other words, it is confirmed whether or not a desired output (para-xylene) is achieved from an input (renewable naphtha raw material) in the case of reaction in the apparatus for pyrolysis in the presence of water vapor (steam cracker) (1), the disproportionation apparatus (2) or the TA apparatus (3), and the para-xylene production apparatus (4). The toluene (b) loaded to the disproportionation apparatus (2) or the TA apparatus (3) in step (W-4) is not illustrated, but is the same as the toluene (b) in step (V-2).

Step (Y-1) is a step of selecting one or more products to be assigned to renewable product(s), in products produced by the para-xylene apparatus.

Step (Y-2) is a step of determining the value of the proportion (P) of the product(s) to be assigned to renewable product(s) in the proportion of the product(s) selected in step (Y-1), with respect to the products obtained by the para-xylene apparatus.

Step (Y-3) is a step of grasping the value of the content rate (Q) of the renewable naphtha contained in the renewable naphtha raw material.

Step (Y-4) is a step of comparing the value of the proportion (P) and the value of the content rate (Q) and confirming that the value of the proportion (P) is equal to or less than the value of the content rate (Q).

Step (Y-1) to step (Y-4) are specifically described with reference to FIG. 10.

Step (Y-1) is to select one or more desired products to be assigned to renewable product(s), in products (for example, para-xylene (e), ortho-xylene (x), meta-xylene (y), and other product (z)) produced by the apparatus to be finally used (here, the para-xylene production apparatus (4)) in the reaction step of providing desired products, as illustrated in FIG. 10A. For example, para-xylene is selected.

Step (Y-2) is to determine the value of the proportion (P) to be assigned as the renewable product(s), to the desired product(s) selected in step (Y-1), in the products produced from the para-xylene production apparatus (4), as illustrated in FIG. 10B. For example, it is assumed as illustrated in FIG. 10B that the products produced from the para-xylene production apparatus (4) are para-xylene (e), ortho-xylene (x), meta-xylene (y) and other product (z) and the respective proportions of the products satisfy para-xylene (e): 80% by mass, ortho-xylene (x): 5% by mass, meta-xylene (y): 10% by mass and other product (z): 5% by mass.

Here, in a case where para-xylene is selected in step (Y-1), the proportion (P1) in 80% by mass of the para-xylene product, to be assigned to renewable benzene, is determined.

Step (Y-3) is to grasp the value of the content rate (Q) of the renewable naphtha (a-1) contained in the renewable naphtha raw material (a), as illustrated in FIG. 10C. For example, as illustrated in FIG. 10C, in a case where the renewable naphtha raw material (a) contains 10% by mass of the renewable naphtha (a-1) and 90% by mass of the petroleum-derived naphtha (a-2), the content rate of the renewable naphtha (a-1) is grasped to be 10% by mass.

Step (Y-4) is to compare the value of the proportion (P) and the value of the content rate (Q) and confirm that the value of the proportion (P) is equal to or less than the value of the content rate (Q), and when the content rate (Q) of the renewable naphtha (a-1) is assumed to be 10% by mass as illustrated in FIG. 10D, the proportion (P), to be assigned to the renewable product(s), to the product(s) is 10% by mass or less.

In a case where two or more products to be assigned to renewable products are selected, the proportion (P) to be assigned means the total value of the proportions to be respectively assigned to the products selected.

In a case where para-xylene is selected in step (Y-1), the proportion (P1) to be assigned to renewable para-xylene is the proportion (P).

In other words, under the assumption that the content rate (Q) of the renewable naphtha (a-1) is 10% by mass, 10% by mass of para-xylene in 80% by mass of the para-xylene product can be assigned to renewable para-xylene in a case where para-xylene is selected as a product to be assigned to a renewable product in step (Y-1). Whether or not the proportion (P1) to be assigned to renewable para-xylene exceeds the content rate (Q) of the renewable naphtha (a-1) (10% by mass in an example of FIG. 10D) is confirmed in step (Y-4).

It is thus possible to assign a value as a renewable product, to the desired product(s) selected in step (Y-1), in products produced from the para-xylene production apparatus (4), depending on the content rate of the renewable naphtha contained in the renewable naphtha raw material.

In other words, with FIG. 10D as an example, it can be determined that (P1) (% by mass) of the para-xylene product in 80% by mass of the para-xylene product corresponds to renewable para-xylene whose 100% corresponds to a renewable component.

According to a third-party certifier, the mass balance system (material balance system) in FIG. 10D indicates a para-xylene product (80 - (P1) (% by mass) of para-xylene product) in which (P1) (% by mass) is subtracted from 80% by mass also actually contains a renewable component, but the para-xylene product is determined not to be handled as renewable para-xylene because a credit of 100% renewable para-xylene is assigned to (P1) (% by mass) of para-xylene.

A value of 10% by mass as the content rate (Q) of the renewable naphtha (a-1) and a value of 80% by mass of the para-xylene product, described in FIG. 10, are values set for convenience in order to facilitate understanding of the present invention, and are not limited to these values, as described above.

### <Management method including step (V) and step (W)>

In a case where step (V) of confirming that xylene and/or benzene are/is produced and step (W) of confirming that para-xylene is produced are included, the following step (V-1), the following step (V-2), the following step (V-3), the following step (W-3), and the following step (W-4) are included. The steps (V-1) to (V-3), and the steps (W-3) to (W-4) are as described in the section <Management method including step (V) and not including step (W)> and the section <Management method including step (W) and not including step (V)>.

In a case where the management method of the present invention includes step (V) and step (W), the management method includes step (Z) of confirming the proportion of a product to which a value as a renewable product is to be assigned.

Step (Z) includes the following step (Z-1), the following step (Z-2), the following step (Z-3), and the following step (Z-4).

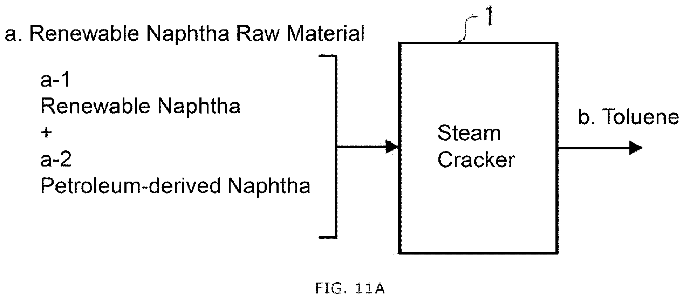

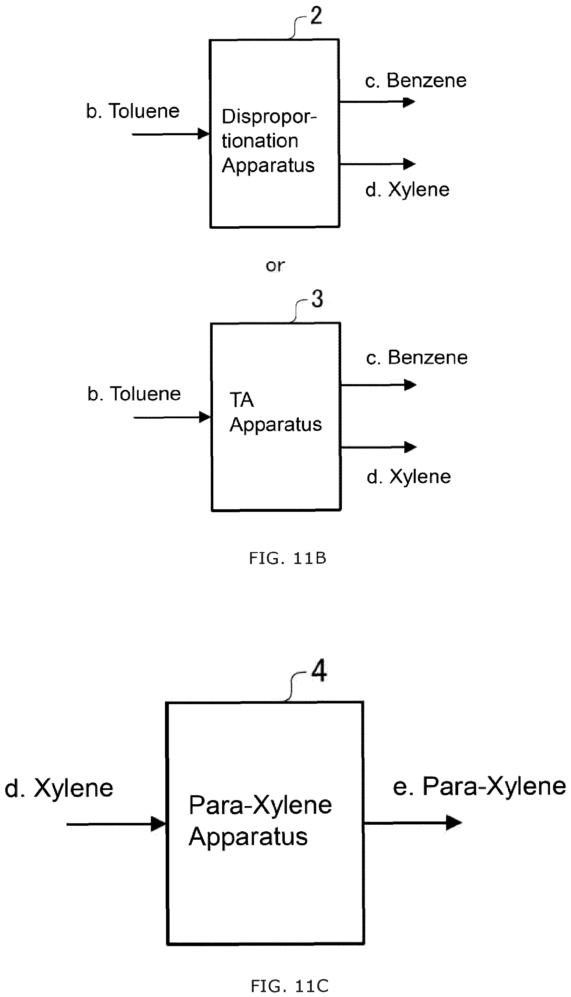

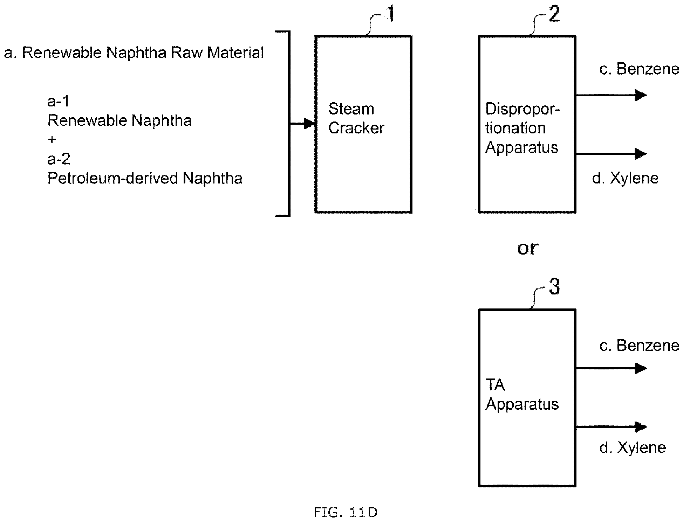

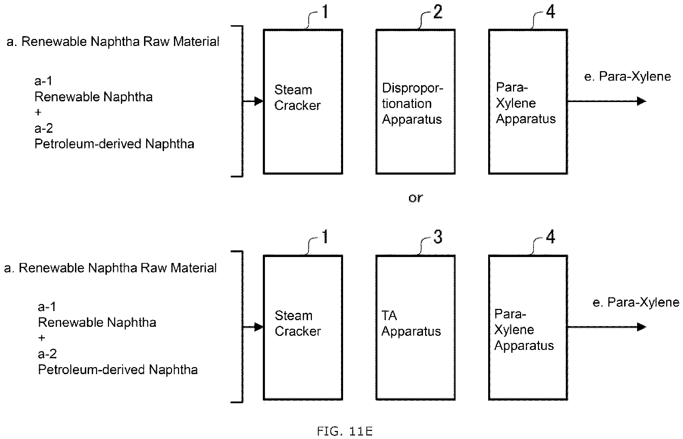

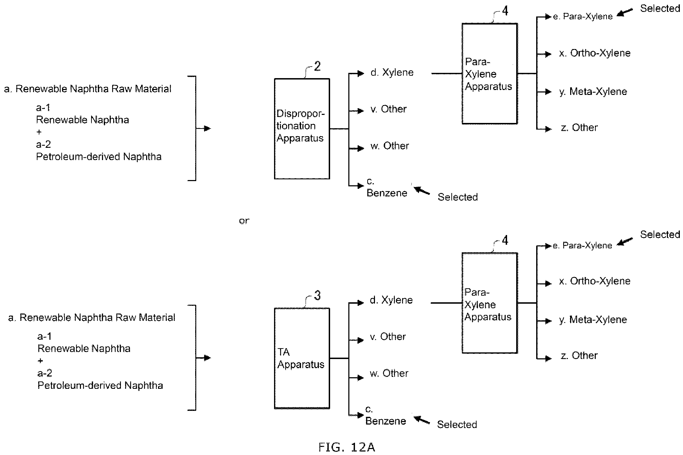

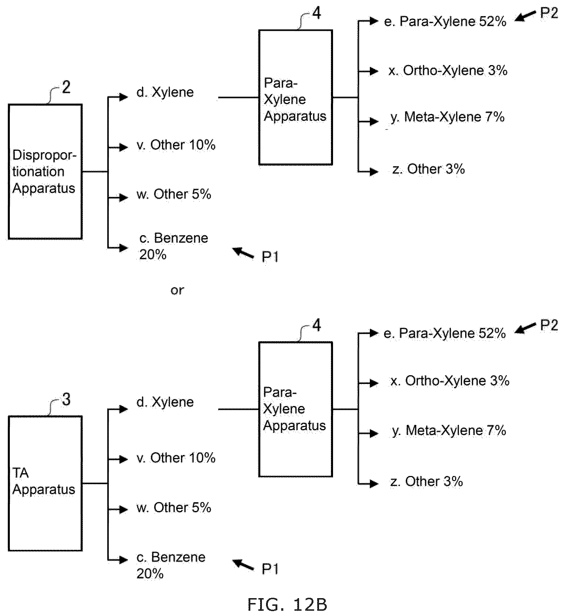

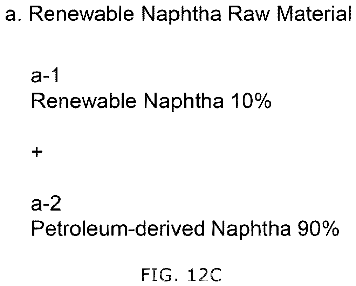

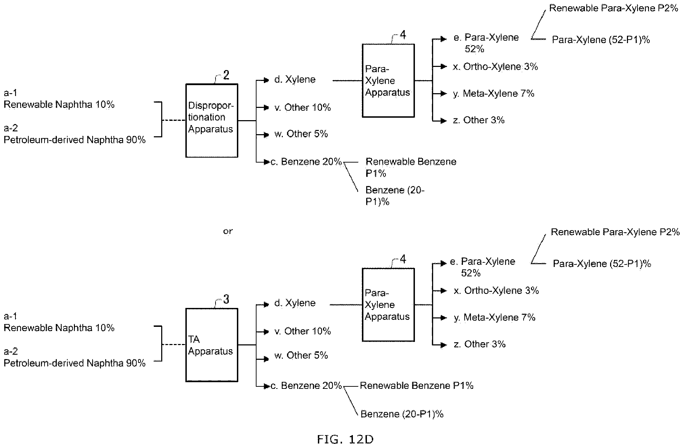

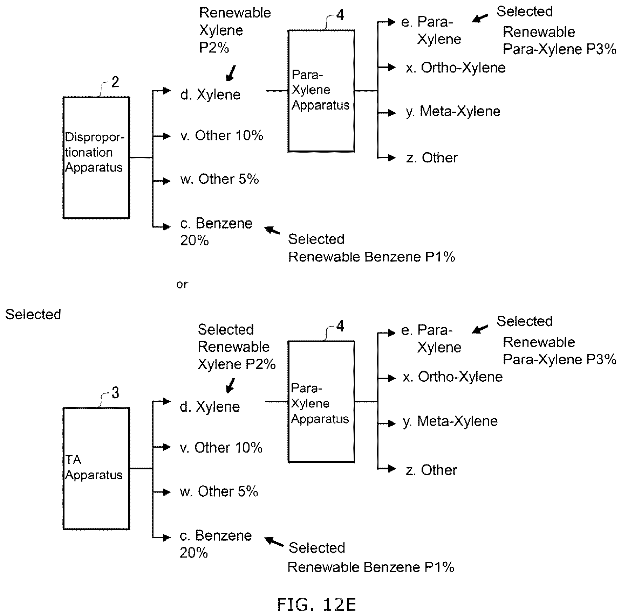

Preferred embodiments of the management method of the present invention, the method including step (V) and step (W), are described with FIG. 11A to FIG. 11E (these are also collectively referred to as "FIG. 11") and FIG. 12A to FIG. 12E (these are also collectively referred to as "FIG. 12").

Step (V-1) is a step of confirming that toluene (b) is produced from the renewable naphtha raw material (a) loaded to the apparatus for pyrolysis in the presence of water vapor (steam cracker) (1), as illustrated in FIG. 11A. In other words, it is confirmed whether or not a desired output (toluene) is achieved from an input (renewable naphtha raw material) in the apparatus for pyrolysis in the presence of water vapor (steam cracker).

Step (V-2) is a step of confirming that xylene (d) and/or benzene (c) are/is produced from toluene (b) loaded to the disproportionation apparatus (2), or taken together with the C9-based component (h) and then loaded to the TA apparatus (3), as illustrated in FIG. 11B. In other words, it is confirmed whether or not a desired output (xylene and/or benzene) is achieved from an input (toluene (optionally including confirmation of an input of the C9-based component)) in the disproportionation apparatus (2) or the TA apparatus (3). The toluene (b) loaded to the disproportionation apparatus (2) or the TA apparatus (3) in step (V-2) may be the toluene (b) obtained in step (V-1), may be other toluene (for example, petroleum-derived toluene), or may be a mixture of the toluene (b) obtained in step (V-1), and other toluene, and is preferably other toluene.

Step (W-3) is a step of confirming that para-xylene (e) is obtained from xylene (d) loaded to the para-xylene production apparatus (4), as illustrated in FIG. 11C. In other words, it is confirmed whether or not a desired output (para-xylene) is achieved from an input (xylene) in the para-xylene production apparatus (4).

Step (V-3) is a step of confirming that xylene (d) and/or benzene (c) are/is produced from the renewable naphtha raw material (a) by treatment in the apparatus for pyrolysis in the presence of water vapor (steam cracker) (1), and the disproportionation apparatus (2) or the TA apparatus (3) in the listed order, as illustrated in FIG. 11D. In other words, it is confirmed whether or not a desired output (xylene and/or benzene) is achieved from an input (renewable naphtha raw material) in the case of reaction in the apparatus for pyrolysis in the presence of water vapor (steam cracker) (1), and the disproportionation apparatus (2) or the TA apparatus (3) in the listed order. The toluene (b) loaded to the disproportionation apparatus (2) or the TA apparatus (3) in step (V-3) is not illustrated, but is the same as the toluene (b) in step (V-2).

Step (W-4) is a step of confirming that para-xylene (e) is obtained from the renewable naphtha raw material (a) by treatment in the apparatus for pyrolysis in the presence of water vapor (steam cracker) (1), the disproportionation apparatus (2) or the TA apparatus (3), and the para-xylene production apparatus (4) in the listed order, as illustrated in FIG. 11E. In other words, it is confirmed whether or not a desired output (para-xylene) is achieved from an input (renewable naphtha raw material) in the case of reaction in the apparatus for pyrolysis in the presence of water vapor (steam cracker) (1), the disproportionation apparatus (2) or the TA apparatus (3), and the para-xylene production apparatus (4) in the listed order. The toluene (b) loaded to the disproportionation apparatus (2) or the TA apparatus (3) in step (W-4) is not illustrated, but is the same as the toluene (b) in step (V-2).

Step (Z-1) is a step of selecting one or more products to be assigned to renewable product(s), in products obtained by the disproportionation apparatus or the TA apparatus, and the para-xylene apparatus.

Step (Z-2) is a step of determining the value of the proportion (P) of the product(s) to be assigned to renewable product(s) in the proportion of the product(s) selected in step (Z-1), with respect to the products obtained by the disproportionation apparatus or the transalkylation (TA) apparatus, and the para-xylene apparatus.

Step (Z-3) is a step of grasping the value of the content rate (Q) of the renewable naphtha contained in the renewable naphtha raw material.

Step (Z-4) is a step of comparing the value of the proportion (P) and the value of the content rate (Q) and confirming that the value of the proportion (P) is equal to or less than the value of the content rate (Q).

Step (Z-1) to step (Z-4) (these steps are also collectively referred to as "step (Z)") are specifically described with reference to FIG. 12.

Step (Z-1) is to select one or more desired products to be assigned to renewable product(s), in products (for example, benzene (c), xylene (d), other product(s) ((v) and/or (w), para-xylene (e), ortho-xylene (x), meta-xylene (y), and other product (z)) produced by the apparatuses to finally used (here, the disproportionation apparatus (2) or the TA apparatus (3), and the para-xylene production apparatus (4)) in the reaction step of providing desired products, as illustrated in FIG. 12A. For example, benzene and para-xylene may be selected, xylene and para-xylene may be selected, or benzene, xylene and para-xylene may be selected.

Step (Z-2) is to determine the value of the proportion (P) to be assigned as the renewable product(s), to the desired product(s) selected in step (Z-1), in the products produced from the disproportionation apparatus (2) or the TA apparatus (3), and the para-xylene production apparatus (4), as illustrated in FIG. 12B. For example, it is assumed as illustrated in FIG. 12B that the products produced from the disproportionation apparatus (2) or the TA apparatus (3) and the para-xylene production apparatus (4) satisfy benzene (c): 20% by mass, other product (v): 10% by mass, other product (w): 5% by mass, para-xylene (e): 52% by mass, ortho-xylene (x): 3% by mass, meta-xylene (y): 7% by mass and other product (z): 3% by mass. There is described for convenience under the assumption that also the products produced from the TA apparatus (3) are those produced at the same product proportions as in the disproportionation apparatus (2). In other words, it is assumed as illustrated in FIG. 12B that the products produced from the TA apparatus (3) satisfy benzene (c): 20% by mass, other product (v): 10% by mass, other product (w): 5% by mass, para-xylene (e): 52% by mass, ortho-xylene (x): 3% by mass, meta-xylene (y): 7% by mass and other product (z): 3% by mass.

Here, in a case where benzene and para-xylene are selected in step (Z-1), the proportion (P1) in 20% by mass of the benzene product, to be assigned to renewable benzene, and the proportion (P2) in 52% by mass of the para-xylene product, to be assigned to renewable para-xylene, are determined.

Step (Z-3) is to grasp the value of the content rate (Q) of the renewable naphtha (a-1) contained in the renewable naphtha raw material (a), as illustrated in FIG. 12C. For example, as illustrated in FIG. 12C, in a case where the renewable naphtha raw material (a) contains 10% by mass of the renewable naphtha (a-1) and 90% by mass of the petroleum-derived naphtha (a-2), the content rate of the renewable naphtha (a-1) is grasped to be 10% by mass.

Step (Z-4) is to compare the value of the proportion (P) and the value of the content rate (Q) and confirm that the value of the proportion (P) is equal to or less than the value of the content rate (Q), and when the content rate (Q) of the renewable naphtha (a-1) is assumed to be 10% by mass as illustrated in FIG. 12D, the proportion (P), to be assigned to the renewable product(s), to the product(s) is 10% by mass or less.

In a case where two or more products to be assigned to renewable products are selected, the proportion (P) to be assigned means the total value of the proportions to be respectively assigned to the products selected.

In a case where two, benzene and para-xylene, are selected in step (Z-1), the total of the proportion (P1) to be assigned to renewable benzene and the proportion (P2) to be assigned to renewable para-xylene is the proportion (P).

In other words, under the assumption that the content rate (Q) of the renewable naphtha (a-1) is 10% by mass, (P1) (% by mass) in 20% by mass of the benzene product and (P2) (% by mass) in 52% by mass of the para-xylene product can be respectively assigned to renewable benzene and to renewable para-xylene in a case where two, benzene and para-xylene, are selected as products to be assigned to renewable products in step (Z-1). For example, in a case where P1 is 5% by mass and P2 is 5% by mass, the total is 10% by mass or less and thus such proportions can be assigned. Alternatively, for example, in a case where P1 is 1% by mass and P2 is 9% by mass, the total is 10% by mass or less and thus such proportions can be assigned. On the other hand, for example, in a case where P1 is 6% by mass and P2 is 7% by mass, the total is 13% by mass and exceeds 10% by mass, and thus such proportions cannot be assigned. Whether or not the total of the proportion (P1) to be assigned to renewable benzene and the proportion (P2) to be assigned to renewable para-xylene exceeds the content rate (Q) of the renewable naphtha (a-1) (10% by mass in an example of FIG. 12D) is confirmed in step (Z-4).

It is thus possible to assign a value as a renewable product, to the desired product(s) selected in step (Z-1), in products produced from the disproportionation apparatus (2) or the TA apparatus (3) and the para-xylene production apparatus (4), depending on the content rate of renewable naphtha contained in the renewable naphtha raw material.

In other words, with FIG. 12D as an example, it can be determined that (P1) (% by mass) of the benzene product in 20% by mass of the benzene product corresponds to renewable benzene whose 100% corresponds to a renewable component and (P2) (% by mass) of the para-xylene product in 52% by mass of the para-xylene product corresponds to renewable para-xylene whose 100% corresponds to a renewable component. The total of (P1) (% by mass) and (P2) (% by mass) is here required not to exceed 10% by mass in FIG. 12D.

According to a third-party certifier, the mass balance system (material balance system) in FIG. 12D indicates that a benzene product (20 - (P1) (% by mass) of benzene product) in which (P1) (% by mass) is subtracted from 20% by mass of the benzene product also actually contains a renewable component, but the benzene product is determined not to be handled as renewable benzene because a credit of 100% renewable benzene is assigned to (P1) (% by mass) of benzene. A para-xylene product (52- (P2) (% by mass) of xylene product) in which (P2) (% by mass) is subtracted from 52% by mass also actually contains a renewable component again, but the para-xylene product is determined not to be handled as renewable para-xylene because a credit of 100% renewable para-xylene is assigned to (P2) (% by mass) of para-xylene.

A value of 10% by mass as the content rate (Q) of the renewable naphtha (a-1), and a value of 20% by mass of the benzene product and a value of 52% by mass of the para-xylene product, described in FIG. 12, are values set for convenience in order to facilitate understanding of the present invention, and are not limited to these values, as described above.

While a case where benzene and para-xylene are selected in step (Z-1) is described as an example in FIG. 12A, for example, xylene can also be selected in step (Z-1).

For example, in a case where xylene and para-xylene are selected in step (Z-1), the total of (P1) (% by mass) of xylene to be allocated as renewable xylene and (P2) (% by mass) of para-xylene to be allocated as renewable para-xylene is required not to exceed 10% by mass. In a case where benzene, xylene and para-xylene are selected in step (Z-1), the total of (P1) (% by mass) of benzene to be allocated as renewable benzene, (P2) (% by mass) of xylene to be allocated as renewable xylene, and (P3) (% by mass) of para-xylene to be allocated as renewable para-xylene is required not to exceed 10% by mass, as illustrated in FIG. 12E.

### (Method for managing terephthalic acid)

It is desired to perform the assignment of a value as a renewable product, with a mass balance system, also to terephthalic acid produced with the method for producing terephthalic acid of the present invention, by a simple and reliable method.

The present invention then provides a management method which, when terephthalic acid is produced with, for example, the method for producing terephthalic acid of the present invention, can assign a value as a renewable product, to such terephthalic acid, depending on the content rate of the renewable naphtha contained in the renewable naphtha raw material, by a simple and reliable method.

The description of the mass balance system is as described above.

The method for managing terephthalic acid of the present invention can be used in production of terephthalic acid with a naphtha raw material containing renewable naphtha.

The management method of the present invention is a method for assigning a value as a renewable product, to the terephthalic acid, depending on the content rate of the renewable naphtha contained in the renewable naphtha raw material.

The management method of the present invention includes step (E) of confirming that the terephthalic acid is obtained, and step (E) includes the following step (V-1), the following step (G-2), the following step (W-3), the following step (E-4), and the following step (E-5).

Step (V-1) is a step of confirming that toluene is produced from the naphtha raw material loaded to an apparatus for pyrolysis in the presence of water vapor (steam cracker).

Step (G-2) is a step of confirming that xylene is produced from toluene loaded to a disproportionation apparatus, or taken together with a C9-based component and then loaded to a transalkylation (TA) apparatus.

Step (W-3) is a step of confirming that para-xylene is obtained from xylene loaded to a para-xylene apparatus.

Step (E-4) is a step of confirming that terephthalic acid is obtained from para-xylene loaded to a terephthalic acid apparatus.

Step (E-5) is a step of confirming that terephthalic acid is obtained from the naphtha raw material by treatment in the apparatus for pyrolysis in the presence of water vapor (steam cracker), the disproportionation apparatus or the transalkylation (TA) apparatus, the para-xylene apparatus, and the terephthalic acid apparatus in the listed order.

Each of the confirmation steps can be performed based on the same consideration as in (Method for managing renewable monocyclic aromatic hydrocarbon) described above.

The method for managing terephthalic acid of the present invention includes step (H) of confirming the proportion of a product to which a value as a renewable product is to be assigned, and step (H) includes the following step (H-1), the following step (H-2), the following step (H-3), and the following step (H-4).

Step (H-1) is a step of selecting a product to be assigned to a renewable product, in products obtained by the terephthalic acid apparatus.

Step (H-2) is a step of determining the value of the proportion (P) of the product to be assigned to a renewable product in the proportion of the product selected in step (H-1), with respect to the products obtained by the terephthalic acid apparatus.

Step (H-3) is a step of grasping the value of the content rate (Q) of the renewable naphtha contained in the naphtha raw material.

Step (H-4) is a step of comparing the value of the proportion (P) and the value of the content rate (Q), and confirming that the value of the proportion (P) is equal to or less than the value of the content rate (Q).

Here, determination and grasping of the value of the proportion (P) to be assigned to a renewable product, and comparison between the value of the proportion (P) and the value of the proportion (Q) can be performed based on the same consideration as in (Method for managing renewable monocyclic aromatic hydrocarbon) described above.

### (Method for managing polyethylene terephthalate (PET))

It is desired to perform the assignment of a value as a renewable product, with a mass balance system, also to PET produced with the production method of the present invention, by a simple and reliable method.

The present invention then provides a management method which, when PET is produced with the method for producing PET of the present invention, can assign a value as a renewable product, to such PET, depending on the content rate of the renewable naphtha contained in the renewable naphtha raw material, by a simple and reliable method.

The description of the mass balance system is as described above.

The method for managing PET of present invention can be used in production of PET with a naphtha raw material containing renewable naphtha.

The management method of the present invention is a method for assigning a value as a renewable product, to the PET, depending on the content rate of the renewable naphtha contained in the naphtha raw material.

The management method of the present invention includes step (F) of confirming that PET is obtained, and step (F) includes the following step (V-1), the following step (G-2), the following step (W-3), the following step (E-4), the following step (F-5), and the following step (F-6).

Step (V-1) is a step of confirming that toluene is produced from the naphtha raw material loaded to an apparatus for pyrolysis in the presence of water vapor (steam cracker).

Step (G-2) is a step of confirming that xylene is produced from toluene loaded to a disproportionation apparatus, or taken together with a C9-based component and then loaded to a transalkylation (TA) apparatus.

Step (W-3) is a step of confirming that para-xylene is obtained from xylene loaded to a para-xylene apparatus.

Step (E-4) is a step of confirming that terephthalic acid is obtained from para-xylene loaded to a terephthalic acid apparatus.

Step (F-5) is a step of confirming that PET is obtained from terephthalic acid loaded to a PET apparatus.

Step (F-6) is a step of confirming that PET is obtained from the naphtha raw material by treatment in the apparatus for pyrolysis in the presence of water vapor (steam cracker), the disproportionation apparatus or the transalkylation (TA) apparatus, the para-xylene apparatus, the terephthalic acid apparatus, and the PET apparatus in the listed order.

Each of the confirmation steps can be performed based on the same consideration as in (Method for managing renewable monocyclic aromatic hydrocarbon) described above.

Furthermore, the method for managing PET of the present invention includes step (J) of confirming the proportion of a product to which a value as a renewable product is to be assigned, and step (J) includes the following step (J-1), the following step (J-2), the following step (J-3), and the following step (J-4).

Step (J-1) is a step of selecting a product to be assigned to a renewable product, in products obtained by the PET apparatus.

Step (J-2) is a step of determining the value of the proportion (P) of the product to be assigned to a renewable product in the proportion of the product selected in step (J-1), with respect to the products obtained by the PET apparatus.

Step (J-3) is a step of grasping the value of the content rate (Q) of the renewable naphtha contained in the naphtha raw material.

Step (J-4) is a step of comparing the value of the proportion (P) and the value of the content rate (Q), and confirming that the value of the proportion (P) is equal to or less than the value of the content rate (Q).

Here, determination and grasping of the value of the proportion (P) to be assigned to a renewable product, and comparison between the value of the proportion (P) and the value of the proportion (Q) can be performed based on the same consideration as in (Method for managing renewable monocyclic aromatic hydrocarbon) described above.

### <Management apparatus, management program>

The management method of the present invention can be implemented with a management apparatus. The processing in each step of the management method to be performed with a management apparatus can be implemented with a computer having a control section constituting the management apparatus.

A management apparatus which implements the management method of the present invention, and a management program (computer program) to be implemented by a computer of the management apparatus are described with, as an example, a case where the management method includes step (V) and does not include step (W). The following description relating to the management apparatus and the management program can be again applied to a case where the management method includes step (W) and does not include step (V), and a case where the management method includes step (V) and step (W).

Examples of a preferred embodiment of the management apparatus of the present invention include the following management apparatus.

"A management apparatus of a renewable monocyclic aromatic hydrocarbon, to be used in production of at least one monocyclic aromatic hydrocarbon of benzene or xylene with a naphtha raw material containing renewable naphtha, wherein
the management apparatus is an apparatus which assigns a value as a renewable product, to the at least one monocyclic aromatic hydrocarbon product, depending on the content rate of the renewable naphtha contained in the naphtha raw material,
the management apparatus includes a confirmation section (I) which confirms that xylene and/or benzene are/is produced,
the confirmation section (I) includes the following unit (V-1), the following unit (V-2), and the following unit (V-3),
the unit (V-1) is a unit which confirms that toluene is produced from the naphtha raw material loaded to an apparatus for pyrolysis in the presence of water vapor (steam cracker),
the unit (V-2) is a unit which confirms that xylene and/or benzene are/is produced from toluene loaded to a disproportionation apparatus or TA apparatus,
the unit (V-3) is a unit which confirms that xylene and/or benzene are/is produced from the naphtha raw material by treatment in the apparatus for pyrolysis in the presence of water vapor (steam cracker), the disproportionation apparatus or the TA apparatus in the listed order,
the management apparatus includes a confirmation section (I) which confirms the proportion of a product to which a value as a renewable product is to be assigned,
the confirmation section (I) includes the following unit (X-1), the following unit (X-2), the following unit (X-3), and the following unit (X-4),
the unit (X-1) is a unit which selects one or more products to be assigned to renewable product(s), in products produced by the disproportionation apparatus or the TA apparatus,
the unit (X-2) is a unit which determines the value of the proportion (P) to be assigned to the renewable product(s), in the proportion of the products selected in the unit (X-1) in the products produced by the disproportionation apparatus or the TA apparatus,
the unit (X-3) is a unit which grasps the value of the content rate (Q) of the renewable naphtha contained in the naphtha raw material, and
the unit (X-4) is a unit which compares the value of the proportion (P) and the value of the content rate (Q) and confirms that the value of the proportion (P) is equal to or less than the value of the content rate (Q)".

Examples of a preferred embodiment of the management program of the present invention include the following management program.

"A management program of a renewable monocyclic aromatic hydrocarbon, to be used in production of at least one monocyclic aromatic hydrocarbon of benzene or xylene with a naphtha raw material containing renewable naphtha, wherein
the management program is a program which assigns a value as a renewable product, to the at least one monocyclic aromatic hydrocarbon product, depending on the content rate of the renewable naphtha contained in the naphtha raw material,
the management program
   (V-1): confirms that toluene is produced from the naphtha raw material loaded to an apparatus for pyrolysis in the presence of water vapor (steam cracker),
   (V-2): confirms that xylene and/or benzene are/is produced from toluene loaded to a disproportionation apparatus or TA apparatus, and
   (V-3): confirms that xylene and/or benzene are/is produced from the naphtha raw material by treatment in the apparatus for pyrolysis in the presence of water vapor (steam cracker), and the disproportionation apparatus or the TA apparatus in the listed order, and
the management program allows a computer to implement processing of
   (X-1): selects one or more products to be assigned to renewable product(s), in products produced by the disproportionation apparatus or the TA apparatus,
   (X-2): determines the value of the proportion (P) to be assigned to the renewable product(s), in the proportion of the products selected in the unit (X-1) in the products produced by the disproportionation apparatus or the TA apparatus,
   (X-3): grasps the value of the content rate (Q) of the renewable naphtha contained in the naphtha raw material, and
   (X-4): compares the value of the proportion (P) and the value of the content rate (Q) and confirms that the value of the proportion (P) is equal to or less than the value of the content rate (Q)".

The above method for managing terephthalic acid of the present invention can also be implemented with a management apparatus. The processing in each step of the management method to be performed with a management apparatus can also be implemented with a computer having a control section constituting the management apparatus.

A management apparatus which implements the method for managing terephthalic acid of the present invention, and a management program (computer program) to be implemented by a computer of the management apparatus are described below.

Examples of a preferred embodiment of the management apparatus of terephthalic acid of the present invention include the following management apparatus.

"A management apparatus of terephthalic acid, to be used in production of terephthalic acid with a naphtha raw material containing renewable naphtha, wherein
the management apparatus is an apparatus which assigns a value as a renewable product, to the terephthalic acid, depending on the content rate of the renewable naphtha contained in the naphtha raw material,
the management apparatus includes a confirmation section (I) which confirms that terephthalic acid is obtained,
the confirmation section (I) includes the following unit (V-1), the following unit (G-2), the following unit (W-3), the following unit (E-4), and the following unit (E-5),
the unit (V-1) is a unit which confirms that toluene is produced from the naphtha raw material loaded to an apparatus for pyrolysis in the presence of water vapor (steam cracker),
the unit (G-2) is a unit which confirms that xylene is produced from toluene loaded to a disproportionation apparatus, or taken together with a C9-based component and then loaded to a transalkylation (TA) apparatus,
the unit (W-3) is a unit which confirms that para-xylene is obtained from xylene loaded to a para-xylene apparatus,
the unit (E-4) is a unit which confirms that terephthalic acid is obtained from para-xylene loaded to a terephthalic acid apparatus,
the unit (E-5) is a unit which confirms that terephthalic acid is obtained from the naphtha raw material by treatment in the apparatus for pyrolysis in the presence of water vapor (steam cracker), the disproportionation apparatus or the transalkylation (TA) apparatus, the para-xylene apparatus, and the terephthalic acid apparatus in the listed order,
the management apparatus includes a confirmation section (I) which confirms the proportion of a product to which a value as a renewable product is to be assigned,
the confirmation section (I) includes the following unit (H-1), the following unit (H-2), the following unit (H-3), and the following unit (H-4),
the unit (H-1) is a unit for selecting a product to be assigned to a renewable product, in products obtained by the terephthalic acid apparatus,
the unit (H-2) is a unit which determines the value of the proportion (P) to be assigned to the renewable product, in the proportion of the product selected in the unit (H-1) in the products obtained by the terephthalic acid apparatus,
the unit (H-3) is a unit which grasps the value of the content rate (Q) of the renewable naphtha contained in the naphtha raw material, and
the unit (H-4) is a unit which compares the value of the proportion (P) and the value of the content rate (Q) and confirms that the value of the proportion (P) is equal to or less than the value of the content rate (Q)".

Examples of a preferred embodiment of the management program of terephthalic acid of the present invention include the following management program.

"A management program of terephthalic acid, to be used in production of terephthalic acid with a naphtha raw material containing renewable naphtha, wherein
the management program is a program which assigns a value as a renewable product, to the terephthalic acid, depending on the content rate of the renewable naphtha contained in the naphtha raw material,
the management program
   (V-1): confirms that toluene is produced from the naphtha raw material loaded to an apparatus for pyrolysis in the presence of water vapor (steam cracker),
   (G-2): confirms that xylene is produced from toluene loaded to a disproportionation apparatus, or taken together with a C9-based component and then loaded to a transalkylation (TA) apparatus,
   (W-3): confirms that para-xylene is obtained from xylene loaded to a para-xylene apparatus,
   (E-4): confirms that terephthalic acid is obtained from para-xylene loaded to a terephthalic acid apparatus, and
   (E-5): confirms that terephthalic acid is obtained from the naphtha raw material by treatment in the apparatus for pyrolysis in the presence of water vapor (steam cracker), the disproportionation apparatus or the transalkylation (TA) apparatus, the para-xylene apparatus, and the terephthalic acid apparatus in the listed order, and
the management program allows a computer to implement processing of
   (H-1): selects a product to be assigned to a renewable product, in products obtained by the terephthalic acid apparatus,
   (H-2): determines the value of the proportion (P) to be assigned to the renewable product, in the proportion of the product selected in step (H-1) in the products produced by the terephthalic acid apparatus,
   (H-3): grasps the value of the content rate (Q) of the renewable naphtha contained in the naphtha raw material, and
   (H-4): compares the value of the proportion (P) and the value of the content rate (Q) and confirms that the value of the proportion (P) is equal to or less than the value of the content rate (Q)".

The above method for managing polyethylene terephthalate of the present invention can also be implemented with a management apparatus. The processing in each step of the management method to be performed with a management apparatus can also be implemented with a computer having a control section constituting the management apparatus.

A management apparatus which implements the method for managing polyethylene terephthalate of the present invention, and a management program (computer program) to be implemented by a computer of the management apparatus are described below.

Examples of a preferred embodiment of the management apparatus of polyethylene terephthalate of the present invention include the following management apparatus.

"A management apparatus of polyethylene terephthalate, to be used in production of polyethylene terephthalate with a naphtha raw material containing renewable naphtha, wherein
the management apparatus is an apparatus which assigns a value as a renewable product, to the polyethylene terephthalate, depending on the content rate of the renewable naphtha contained in the naphtha raw material,
the management apparatus includes a confirmation section (I) which confirms that polyethylene terephthalate is obtained,
the confirmation section (I) includes the following unit (V-1), the following unit (G-2), the following unit (W-3), the following unit (E-4), the following unit (F-5), and the following unit (F-6),
the unit (V-1) is a unit which confirms that toluene is produced from the naphtha raw material loaded to an apparatus for pyrolysis in the presence of water vapor (steam cracker),
the unit (G-2) is a unit which confirms that xylene is produced from toluene loaded to a disproportionation apparatus, or taken together with a C9-based component and then loaded to a transalkylation (TA) apparatus,
the unit (W-3) is a unit which confirms that para-xylene is obtained from xylene loaded to a para-xylene apparatus,
the unit (E-4) is a unit which confirms that terephthalic acid is obtained from para-xylene loaded to a terephthalic acid apparatus,
the unit (F-5) is a unit which confirms that polyethylene terephthalate is obtained from terephthalic acid loaded to a PET apparatus, and
the unit (F-6) is a unit which confirms that polyethylene terephthalate is obtained from the naphtha raw material by treatment in the apparatus for pyrolysis in the presence of water vapor (steam cracker), the disproportionation apparatus or the transalkylation (TA) apparatus, the para-xylene apparatus, the terephthalic acid apparatus, and the PET apparatus in the listed order,
the management apparatus includes a confirmation section (I) which confirms the proportion of a product to which a value as a renewable product is to be assigned,
the confirmation section (I) includes the following unit (J-1), the following unit (J-2), the following unit (J-3), and the following unit (J-4),
the unit (J-1) is a unit which selects a product to be assigned to a renewable product, in products produced by the PET apparatus,
the unit (J-2) is a unit which determines the value of the proportion (P) to be assigned to the renewable product, in the proportion of the product selected in the unit (J-1) in the products obtained by the PET apparatus,
the unit (J-3) is a unit which grasps the value of the content rate (Q) of the renewable naphtha contained in the naphtha raw material, and
the unit (J-4) is a unit which compares the value of the proportion (P) and the value of the content rate (Q) and confirms that the value of the proportion (P) is equal to or less than the value of the content rate (Q)".

Examples of a preferred embodiment of the management program of polyethylene terephthalate of the present invention include the following management program.

"A management program of polyethylene terephthalate, to be used in production of polyethylene terephthalate with a naphtha raw material containing renewable naphtha, wherein
the management program is a program which assigns a value as a renewable product, to the polyethylene terephthalate, depending on the content rate of the renewable naphtha contained in the naphtha raw material,
the management program
   (V-1): confirms that toluene is produced from the naphtha raw material loaded to an apparatus for pyrolysis in the presence of water vapor (steam cracker),
   (G-2): confirms that xylene is produced from toluene loaded to a disproportionation apparatus, or taken together with a C9-based component and then loaded to a transalkylation (TA) apparatus,
   (W-3): confirms that para-xylene is obtained from xylene loaded to a para-xylene apparatus,
   (E-4): confirms that terephthalic acid is obtained from para-xylene loaded to a terephthalic acid apparatus,
   (F-5): confirms that polyethylene terephthalate is obtained from terephthalic acid loaded to a PET apparatus, and
   (F-6): confirms that polyethylene terephthalate is obtained from the naphtha raw material by treatment in the apparatus for pyrolysis in the presence of water vapor (steam cracker), the disproportionation apparatus or the transalkylation (TA) apparatus, the para-xylene apparatus, the terephthalic acid apparatus, and the PET apparatus in the listed order, and
the management program allows a computer to implement processing of
   (J-1): selects a product to be assigned to a renewable product in products obtained by the PET apparatus,
   (J-2): determines the value of the proportion (P) to be assigned to the renewable product, in the proportion of the product selected in step (J-1) in the products obtained by the PET apparatus,
   (J-3): grasps the value of the content rate (Q) of the renewable naphtha contained in the naphtha raw material, and
   (J-4): compares the value of the proportion (P) and the value of the content rate (Q) and confirms that the value of the proportion (P) is equal to or less than the value of the content rate (Q)".

### <<Management apparatus>>

The management apparatus is an apparatus which implements the management method of the present invention.

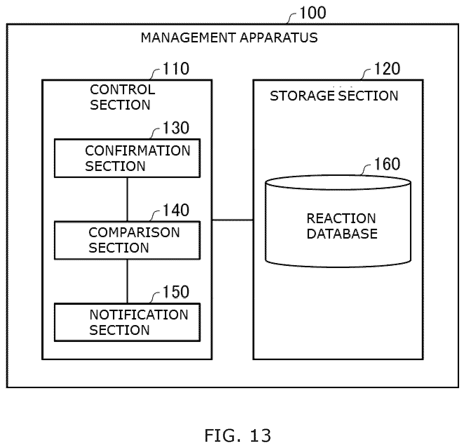

A preferred embodiment of the management apparatus is described with reference to FIG. 13.

A management apparatus 100 includes a control section 110 and a storage section 120.

The control section 110 includes a confirmation section (I) 130, a comparison section 140, and a notification section (output section) 150, and the storage section 120 includes a reaction database 160.

A hardware configuration and a function configuration of management apparatus 100 are described.

### <<Hardware configuration of management apparatus>>

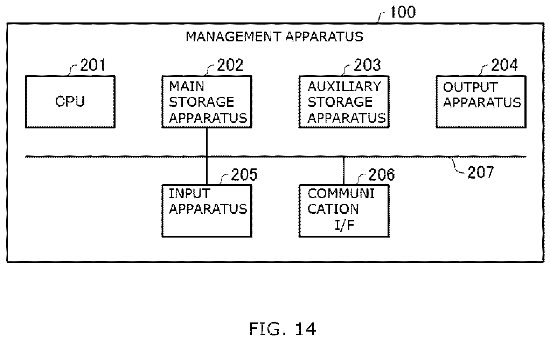

FIG. 14 is a block diagram illustrating one example of a hardware configuration of a management apparatus 100.

As illustrated in FIG. 14, the management apparatus 100 includes each of the following sections. These sections are each connected via a bus 207.

CPU 201 is a processing apparatus (computer) which performs various control and computing. The CPU 201 realizes various functions by implementing OS and a computer program stored in a main storage apparatus 202 or the like. In other words, the CPU 201 in the present embodiment is configured to implement a management program and thus function as the control section 110 of the management apparatus, to implement the management method.

The CPU 201 controls the operation of the entire management apparatus 100. The apparatus which controls the operation of the entire management apparatus 100, while is the CPU 201 in the present embodiment, is not limited thereto, and may be, for example, FPGA (Field Programmable Gate Array).

The management program and various databases are not necessarily stored in the main storage apparatus 202, an auxiliary storage apparatus 203, or the like. The management program and various databases may be stored in other information processing apparatus to be connected to the management apparatus 100 via the internet, LAN (Local Area Network), WAN (Wide Area Network), or the like. The management program and various databases may also be acquired from such other information processing apparatus by the management apparatus 100, and then implemented.

The main storage apparatus 202 is a computer-readable storage medium which stores various programs and stores data necessary for implementing various programs.

The main storage apparatus 202 includes ROM and RAM not illustrated.

ROM stores various programs such as BIOS.

RAM functions as an operation range that is extended during the implementation of various programs stored in ROM by the CPU 201. RAM is not restricted and can be appropriately selected depending on the object. Examples of RAM include DRAM and SRAM.

The auxiliary storage apparatus 203 is not particularly restricted as long as it can store a variety of information, and can be appropriately selected depending on the object, and examples thereof include a solid-state drive and a hard disk drive. The auxiliary storage apparatus 203 may be, for example, a transportable storage apparatus such as a CD drive, a DVD drive, or a BD drive.

An output apparatus 204 here used can be a display, a speaker, or the like. The display is not particularly restricted, one known can be appropriately used, and examples thereof include a liquid crystal display and an organic EL display.

An input apparatus 205 is not particularly restricted as long as it can receive various requirements for the management apparatus 100, one known can be appropriately used, and examples thereof include a keyboard and a touch panel.

A communication interface (communication I/F) 206 is not particularly restricted, one known can be appropriately used, and examples thereof include a wireless or wired communication device.

The above hardware configuration enables a processing function of the management apparatus 100 to be realized.

### <<Function configuration of management apparatus>>

Returning to FIG. 13, the management apparatus 100 includes a control section 110 and a storage section 120. The control section 110 controls the entire management apparatus 100.

The control section 110 includes a confirmation section (I) 130, a comparison section 140, and a notification section (output section) 150.

The confirmation section (I) of the control section 110 performs confirmation operations described in the units (V-1) to (V-3).

The confirmation section (I) of the control section 110 also performs confirmation operations which include confirming that a product to be assigned to a renewable product is selected in the unit (X-1), confirming that the value of the proportion (P) to be assigned to the renewable product in the product selected in the unit (X-2) is set, confirming the value of the content rate (Q) of the renewable naphtha in the unit (X-3), and comparing the value of the proportion (P) and the value of the content rate (Q) and confirming that the value of the proportion (P) is equal to or less than the value of the content rate (Q) in the unit (X-4).

The comparison section 140 of the control section 110 performs a comparison operation for comparing the value of the proportion (P) and the value of the content rate (Q) in order to make confirmation by the confirmation section (I) in the unit (X-4).

The notification section 150 of the control section 110 notifies (output) that (P) (% by mass) of the selected product can be assigned to the renewable product in a case where the value of the proportion (P) is equal to or less than the value of the content rate (Q), whereas notifies (output) that the value of the proportion (P) exceeds the value of the content rate (Q) in a case where the value of the proportion (P) exceeds the value of the content rate (Q).

In other words, the management apparatus 100 is such that, after the management method is implemented, a result which is acquired by the management method and to which a value as a renewable product to a product selected depending on the content rate of the renewable naphtha contained in the naphtha raw material is assigned is output.

The reaction database 160 in the storage section 120 of the control section 110 stores information on an apparatus used in the management method of the present invention, and information on reaction to be performed in the apparatus. In other words, the storage section 120 is provided with a storage medium which is a computer-readable storage medium in which a computer program is stored, and which stores a management program which allows the management method to be implemented by the control section 110 including a computer.

The amount of production and the yield of each product obtained from each of the apparatuses can be determined by measurement, and not only the yield results are obtained by actual measurement, but also can be determined according to theoretical calculation or predicted based on the previous data by use of the reaction database 160.

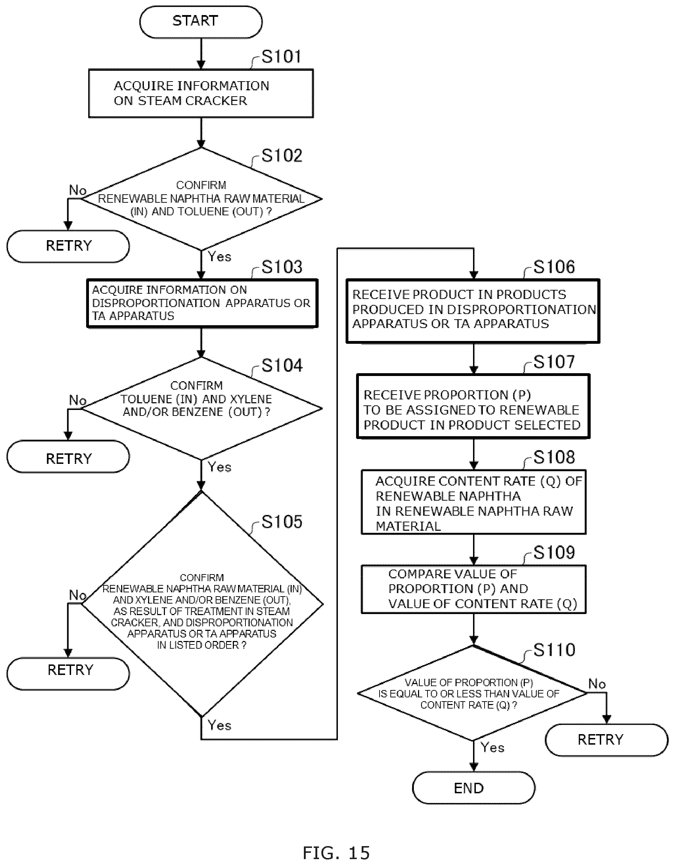

Next, a processing procedure of the management program is described. FIG. 15 is a flowchart illustrating one example of a processing procedure of a management program in the control section 110 of the management apparatus 100. Hereinafter, the processing procedure is described with reference to FIG. 15.

In step S101, the confirmation section 130 of the control section 110 of the management apparatus 100 acquires information on the steam cracker, and transfers the processing to step S102.

In step S102, the confirmation section 130 of the control section 110 of the management apparatus 100 confirms that toluene (OUT) is produced from a renewable naphtha raw material (IN) in the steam cracker, and transfers the processing to step S103 in a case where such production is confirmed.

In step S103, the confirmation section 130 of the control section 110 of the management apparatus 100 acquires information on the disproportionation apparatus or the TA apparatus, and transfers the processing to step S104.

In step S104, the confirmation section 130 of the control section 110 of the management apparatus 100 confirms that xylene and/or benzene (OUT) are/is produced from toluene (IN) in the disproportionation apparatus or the TA apparatus, and transfers the processing to step S105 in a case where such production is confirmed.

In step S105, the confirmation section 130 of the control section 110 of the management apparatus 100 confirms that treatment is performed in the steam cracker, and the disproportionation apparatus or the TA apparatus in the listed order and confirms that the treatment results in production of xylene and/or benzene (OUT) from renewable naphtha raw material (IN), and transfers the processing to step S106 in a case where such production is confirmed.

In step S106, the confirmation section 130 of the control section 110 of the management apparatus 100 receives information on the assignment to a renewable product in products produced in the disproportionation apparatus or the TA apparatus, selected by an operator, from an input apparatus (input apparatus 205 in FIG. 14) in the management apparatus 100, and transfers the processing to step S107.

In step S107, the confirmation section 130 of the control section 110 of the management apparatus 100 receives the value of the proportion (P) to be assigned to a renewable product, in the production of the product selected, set by the operator, from the input apparatus (input apparatus 205 in FIG. 14) in the management apparatus 100, and transfers the processing to step S108.

In step S108, the confirmation section 130 of the control section 110 of the management apparatus 100 acquires the value of the content rate (Q) of the renewable naphtha in the renewable naphtha raw material loaded to the steam cracker, and transfers the processing to step S109.

In step S109, the comparison section 140 of the control section 110 of the management apparatus 100 compares the value of the proportion (P) and the value of the content rate (Q), and transfers the processing to step S110.

In step S110, the confirmation section 130 of the control section 110 of the management apparatus 100 confirms whether or not the value of the proportion (P) is equal to or less than the value of the content rate (Q) based on the result of comparison between the value of the proportion (P) and the value of the content rate (Q) performed in the comparison section 140 of the control section 110 of the management apparatus 100, and ends the processing in a case where the value of the proportion (P) is equal to or less than the value of the content rate (Q) is confirmed.

The processing can be ended to assign a value as a renewable product, to a desired product selected, depending on the content rate of renewable naphtha contained in the renewable naphtha raw material. The assignment result is notified to a user via the notification section 150 of the control section 110 of the management apparatus 100. In other words, as described above, the management apparatus 100 is such that, after the management method is implemented, a result which is acquired by the management method and to which a value as a renewable product to a product selected depending on the content rate of the renewable naphtha contained in the naphtha raw material is assigned is output.

In a case where the conditions are not met during the processing, such no meeting is notified to the user via, for example, the notification section 150 of the control section 110 of the management apparatus 100. In this case, the operator can review the type of a product to be selected in the (X-1), can review the value of the proportion (P) to be assigned to the product selected, can review the value of the content rate (Q) of the renewable naphtha, and/or furthermore can review various conditions such as reaction conditions and try the processing again.

While the management method including step (V) and not including step (W) is taken as an example and the management apparatus and the management program with the management method are described in FIGS. 13 to 15, the same management apparatus and management program can be applied to not only the management method including step (W) and not including step (V), but also the management method including step (V) and step (W).

### (First variation)

### (Function configuration of management apparatus implementing method for managing terephthalic acid)

The function configuration of a management apparatus which implements the above method for managing terephthalic acid also has, in its basic portion, the same configuration as in the management apparatus 100 illustrated in FIG. 13 and FIG. 14. With reference to FIG. 13 and FIG. 14, the function configuration of the management apparatus which implements the method for managing terephthalic acid is described.

As illustrated in FIG. 13, a management apparatus 100 which implements the method for managing terephthalic acid also includes a control section 110 and a storage section 120. The control section 110 controls the entire management apparatus 100.

The control section 110 includes a confirmation section (I) 130, a comparison section 140, and a notification section (output section) 150.

The confirmation section (I) of the control section 110 performs confirmation operations described in the above-mentioned unit (V-1), unit (G-2), unit (W-3), unit (E-4), and unit (E-5).

The confirmation section (I) of the control section 110 performs confirmation operations which include confirming that a product to be assigned to a renewable product, in products obtained by the terephthalic acid apparatus, is selected in the unit (H-1), confirming that the value of the proportion (P) to be assigned to the renewable product, in the proportion of the product selected in the unit (H-1) in the products obtained by the terephthalic acid apparatus, is set in the unit (H-2), confirming the value of the content rate (Q) of the renewable naphtha contained in the naphtha raw material in the unit (H-3), and comparing the value of the proportion (P) and the value of the content rate (Q) and confirming that the value of the proportion (P) is equal to or less than the value of the content rate (Q) in the unit (H-4).

The comparison section 140 of the control section 110 performs a comparison operation for comparing the value of the proportion (P) and the value of the content rate (Q) in order to make confirmation by the confirmation section (I) in the unit (H-4).

The notification section 150 of the control section 110 notifies (output) that (P) (% by mass) of the selected product can be assigned to the renewable product in a case where the value of the proportion (P) is equal to or less than the value of the content rate (Q), whereas notifies (output) that the value of the proportion (P) exceeds the value of the content rate (Q) in a case where the value of the proportion (P) exceeds the value of the content rate (Q).

In other words, the management apparatus 100 which implements the method for managing terephthalic acid is such that, after the method for managing terephthalic acid is implemented, a result which is acquired by the method for managing terephthalic acid and to which a value as a renewable product to a product selected depending on the content rate of the renewable naphtha contained in the naphtha raw material is assigned is output.

The reaction database 160 in the storage section 120 of the control section 110 stores information on an apparatus used in the method for managing terephthalic acid of the present invention, and information on reaction to be performed in the apparatus. In other words, the storage section 120 is provided with a storage medium which is a computer-readable storage medium in which a computer program is stored, and which stores a management program which allows the method for managing terephthalic acid to be implemented by the control section 110 including a computer.

The amount of production and the yield of each product obtained from each of the apparatuses can be determined by measurement, and not only the yield results are obtained by actual measurement, but also can be determined according to theoretical calculation or predicted based on the previous data by use of the reaction database 160.

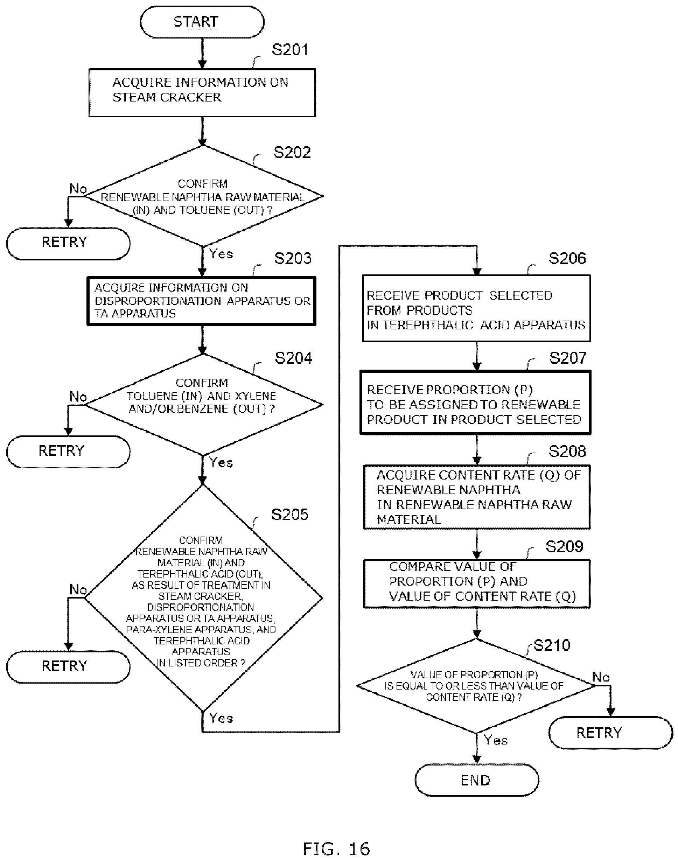

Next, a processing procedure of the management program of terephthalic acid is described. FIG. 16 is a flowchart illustrating one example of a processing procedure of a management program of terephthalic acid in the control section 110 of the management apparatus 100 of terephthalic acid. Hereinafter, the processing procedure is described with reference to FIG. 16.

In step S201, the confirmation section 130 of the control section 110 of the management apparatus 100 of terephthalic acid acquires information on the steam cracker, and transfers the processing to step S202.

In step S202, the confirmation section 130 of the control section 110 of the management apparatus 100 of terephthalic acid confirms that toluene (OUT) is produced from a renewable naphtha raw material (IN) in the steam cracker, and transfers the processing to step S203 in a case where such production is confirmed.

In step S203, the confirmation section 130 of the control section 110 of the management apparatus 100 of terephthalic acid acquires information on the disproportionation apparatus or the TA apparatus, and transfers the processing to step S204.

In step S204, the confirmation section 130 of the control section 110 of the management apparatus 100 of terephthalic acid confirms that xylene and/or benzene (OUT) are/is produced from toluene (IN) in the disproportionation apparatus or the TA apparatus, and transfers the processing to step S205 in a case where such production is confirmed.

In step S205, the confirmation section 130 of the control section 110 of the management apparatus 100 of terephthalic acid confirms that treatment is performed in the steam cracker, the disproportionation apparatus or the TA apparatus, the para-xylene apparatus, and the terephthalic acid apparatus in the listed order and confirms that the treatment results in production of terephthalic acid (OUT) from renewable naphtha raw material (IN), and transfers the processing to step S206 in a case where such production is confirmed.

In step S206, the confirmation section 130 of the control section 110 of the management apparatus 100 of terephthalic acid receives information on the assignment to a renewable product in products produced in the terephthalic acid apparatus, selected by an operator, from an input apparatus (input apparatus 205 in FIG. 14) in the management apparatus 100, and transfers the processing to step S207.

In step S207, the confirmation section 130 of the control section 110 of the management apparatus 100 of terephthalic acid receives the value of the proportion (P) to be assigned to a renewable product, in the production of the product selected, set by the operator, from the input apparatus (input apparatus 205 in FIG. 14) in the management apparatus 100 of terephthalic acid, and transfers the processing to step S208.

In step S208, the confirmation section 130 of the control section 110 of the management apparatus 100 of terephthalic acid acquires the value of the content rate (Q) of the renewable naphtha in the renewable naphtha raw material loaded to the steam cracker, and transfers the processing to step S209.

In step S209, the comparison section 140 of the control section 110 of the management apparatus 100 of terephthalic acid compares the value of the proportion (P) and the value of the content rate (Q), and transfers the processing to step S210.

In step S210, the confirmation section 130 of the control section 110 of the management apparatus 100 of terephthalic acid confirms whether or not the value of the proportion (P) is equal to or less than the value of the content rate (Q) based on the result of comparison between the value of the proportion (P) and the value of the content rate (Q) performed in the comparison section 140 of the control section 110 of the management apparatus 100 of terephthalic acid, and ends the processing in a case where the value of the proportion (P) is equal to or less than the value of the content rate (Q) is confirmed.

The processing can be ended to assign a value as a renewable product, to a desired product selected, depending on the content rate of renewable naphtha contained in the renewable naphtha raw material. The assignment result is notified to a user via the notification section 150 of the control section 110 of the management apparatus 100 of terephthalic acid. In other words, as described above, the management apparatus 100 of terephthalic acid is such that, after the method for managing terephthalic acid is implemented, a result which is acquired by the method for managing terephthalic acid and to which a value as a renewable product to a product selected depending on the content rate of the renewable naphtha contained in the naphtha raw material is assigned is output.

In a case where the conditions are not met during the processing, such no meeting is notified to the user via, for example, the notification section 150 of the control section 110 of the management apparatus 100 of terephthalic acid. In this case, the operator can review the type of a product to be selected in the (X-1), can review the value of the proportion (P) to be assigned to the product selected, can review the value of the content rate (Q) of the renewable naphtha, and/or furthermore can review various conditions such as reaction conditions and try the processing again.

### (Second variation)

### (Function configuration of management apparatus implementing method for managing polyethylene terephthalate)

The function configuration of a management apparatus which implements the above method for managing polyethylene terephthalate also has, in its basic portion, the same configuration as in the management apparatus 100 illustrated in FIG. 13 and FIG. 14. With reference to FIG. 13 and FIG. 14, the function configuration of the management apparatus which implements the method for managing polyethylene terephthalate is described.

As illustrated in FIG. 13, a management apparatus 100 which implements the method for managing polyethylene terephthalate also includes a control section 110 and a storage section 120. The control section 110 controls the entire management apparatus 100.

The control section 110 includes a confirmation section (I) 130, a comparison section 140, and a notification section (output section) 150.

The confirmation section (I) of the control section 110 performs confirmation operations described in the above-mentioned unit (V-1), unit (G-2), unit (W-3), unit (E-4), unit (F-5), and unit (F-6).

The confirmation section (I) of the control section 110 performs confirmation operations which confirm that a product to be assigned to a renewable product, in products obtained by the PET apparatus, is selected in the unit (J-1), confirming that the value of the proportion (P) to be assigned to the renewable product, in the proportion of the product selected in the unit (J-1) in the products obtained by the PET apparatus, is set in the unit (J-2), confirming the value of the content rate (Q) of the renewable naphtha contained in the naphtha raw material in the unit (J-3), and comparing the value of the proportion (P) and the value of the content rate (Q) and confirming that the value of the proportion (P) is equal to or less than the value of the content rate (Q) in the unit (J-4).

The comparison section 140 of the control section 110 performs a comparison operation for comparing the value of the proportion (P) and the value of the content rate (Q) in order to make confirmation by the confirmation section (I) in the unit (J-4).

The notification section 150 of the control section 110 notifies (output) that (P) (% by mass) of the selected product can be assigned to the renewable product in a case where the value of the proportion (P) is equal to or less than the value of the content rate (Q), whereas notifies (output) that the value of the proportion (P) exceeds the value of the content rate (Q) in a case where the value of the proportion (P) exceeds the value of the content rate (Q).

In other words, the management apparatus 100 which implements the method for managing polyethylene terephthalate is such that, after the method for managing polyethylene terephthalate is implemented, a result which is acquired by the method for managing polyethylene terephthalate and to which a value as a renewable product to a product selected depending on the content rate of the renewable naphtha contained in the naphtha raw material is assigned is output.

The reaction database 160 in the storage section 120 of the control section 110 stores information on an apparatus used in the method for managing polyethylene terephthalate of the present invention, and information on reaction to be performed in the apparatus. In other words, the storage section 120 is provided with a storage medium which is a computer-readable storage medium in which a computer program is stored, and which stores a management program which allows the method for managing polyethylene terephthalate to be implemented by the control section 110 including a computer.

The amount of production and the yield of each product obtained from each of the apparatuses can be determined by measurement, and not only the yield results are obtained by actual measurement, but also can be determined according to theoretical calculation or predicted based on the previous data by use of the reaction database 160.

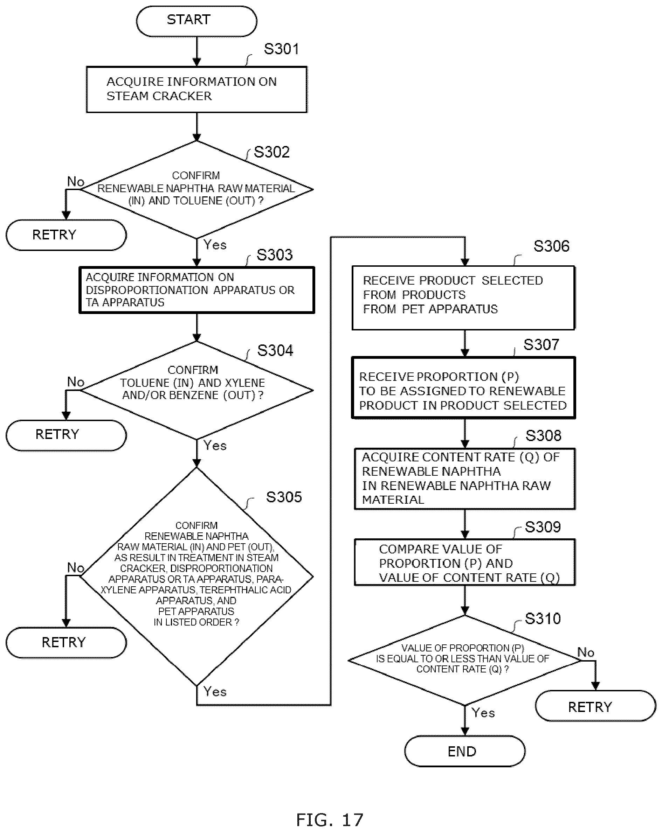

Next, a processing procedure of the management program of polyethylene terephthalate is described. FIG. 17 is a flowchart illustrating one example of a processing procedure of a management program of polyethylene terephthalate in the control section 110 of the management apparatus 100 of polyethylene terephthalate. Hereinafter, the processing procedure is described with reference to FIG. 17.

In step S301, the confirmation section 130 of the control section 110 of the management apparatus 100 of polyethylene terephthalate acquires information on the steam cracker, and transfers the processing to step S302.

In step S302, the confirmation section 130 of the control section 110 of the management apparatus 100 of polyethylene terephthalate confirms that toluene (OUT) is produced from a renewable naphtha raw material (IN) in the steam cracker, and transfers the processing to step S303 in a case where such production is confirmed.

In step S303, the confirmation section 130 of the control section 110 of the management apparatus 100 of polyethylene terephthalate acquires information on the disproportionation apparatus or the TA apparatus, and transfers the processing to step S304.

In step S304, the confirmation section 130 of the control section 110 of the management apparatus 100 of polyethylene terephthalate confirms that xylene and/or benzene (OUT) are/is produced from toluene (IN) in the disproportionation apparatus or the TA apparatus, and transfers the processing to step S305 in a case where such production is confirmed.

In step S305, the confirmation section 130 of the control section 110 of the management apparatus 100 of polyethylene terephthalate confirms that treatment is performed in the steam cracker, the disproportionation apparatus or the TA apparatus, the para-xylene apparatus, the terephthalic acid apparatus, and the PET apparatus in the listed order and confirms that the treatment results in production of terephthalic acid (OUT) from renewable naphtha raw material (IN), and transfers the processing to step S306 in a case where such production is confirmed.

In step S306, the confirmation section 130 of the control section 110 of the management apparatus 100 of polyethylene terephthalate receives information on the assignment to a renewable product in products produced in the PET apparatus, selected by an operator, from an input apparatus (input apparatus 205 in FIG. 14) in the management apparatus 100, and transfers the processing to step S307.

In step S307, the confirmation section 130 of the control section 110 of the management apparatus 100 of polyethylene terephthalate receives the value of the proportion (P) to be assigned to a renewable product, in the production of the product selected, set by the operator, from the input apparatus (input apparatus 205 in FIG. 14) in the management apparatus 100 of polyethylene terephthalate, and transfers the processing to step S308.

In step S308, the confirmation section 130 of the control section 110 of the management apparatus 100 of polyethylene terephthalate acquires the value of the content rate (Q) of the renewable naphtha in the renewable naphtha raw material loaded to the steam cracker, and transfers the processing to step S309.

In step S309, the comparison section 140 of the control section 110 of the management apparatus 100 of polyethylene terephthalate compares the value of the proportion (P) and the value of the content rate (Q), and transfers the processing to step S310.

In step S310, the confirmation section 130 of the control section 110 of the management apparatus 100 of polyethylene terephthalate confirms whether or not the value of the proportion (P) is equal to or less than the value of the content rate (Q) based on the result of comparison between the value of the proportion (P) and the value of the content rate (Q) performed in the comparison section 140 of the control section 110 of the management apparatus 100 of polyethylene terephthalate, and ends the processing in a case where the value of the proportion (P) is equal to or less than the value of the content rate (Q) is confirmed.

The processing can be ended to assign a value as a renewable product, to a desired product selected, depending on the content rate of renewable naphtha contained in the renewable naphtha raw material. The assignment result is notified to a user via the notification section 150 of the control section 110 of the management apparatus 100 of polyethylene terephthalate. In other words, as described above, the management apparatus 100 of polyethylene terephthalate is such that, after the method for managing polyethylene terephthalate is implemented, a result which is acquired by the method for managing polyethylene terephthalate and to which a value as a renewable product to a product selected depending on the content rate of the renewable naphtha contained in the naphtha raw material is assigned is output.

In a case where the conditions are not met during the processing, such no meeting is notified to the user via, for example, the notification section 150 of the control section 110 of the management apparatus 100 of polyethylene terephthalate. In this case, the operator can review the type of a product to be selected in the (X-1), can review the value of the proportion (P) to be assigned to the product selected, can review the value of the content rate (Q) of the renewable naphtha, and/or furthermore can review various conditions such as reaction conditions and try the processing again.

While some embodiments of the present invention are described, these embodiments are illustrative and are not intended to limit the scope of the invention. These embodiments can be carried out in other various modes, and can be variously omitted, replaced, and/or modified without departing from the gist of the invention. These embodiments and variations thereof are not only included in the scope and/or gist of the invention, but also included in the scopes of the invention recited in claims and equivalents thereof.

## Claims

1. A method for producing at least one monocyclic aromatic hydrocarbon of benzene or xylene with a naphtha raw material containing renewable naphtha, the method comprising:
step (A-1) of pyrolyzing the naphtha raw material in the presence of water vapor, to produce and separate toluene; and
step (A-2) of subjecting toluene to disproportionation reaction or transalkylation reaction, to produce and separate at least one monocyclic aromatic hydrocarbon of benzene or xylene.

2. The production method according to claim 1, wherein toluene is produced and separated by distillation or extraction of a component containing a monocyclic aromatic hydrocarbon, in a pyrolysis product obtained by pyrolyzing the naphtha raw material in the presence of water vapor, in the step (A-1).

3. The production method according to claim 1, further comprising step (A-3) of subjecting xylene obtained in the step (A-2), to adsorption separation or crystallization separation, to separate para-xylene.

4. The production method according to claim 3, comprising a step of subjecting a residue containing at least one of ortho-xylene or meta-xylene after separation of para-xylene in the step (A-3), to isomerization treatment, to produce para-xylene, and subsequently separating the para-xylene by adsorption separation or crystallization separation.

5. The production method according to any one of claims 1 to 4, wherein the monocyclic aromatic hydrocarbon contains a radioactive carbon atom ¹⁴C.

6. Xylene containing a radioactive carbon atom ¹⁴C.

7. The xylene according to claim 6, wherein the radioactive carbon atom ¹⁴C is derived from bio-naphtha.

8. A method for producing terephthalic acid with a naphtha raw material containing renewable naphtha, the method comprising:
step (A-3) of obtaining para-xylene by the method according to claim 3; and
step (A-4) of oxidizing the para-xylene, to obtain terephthalic acid.

9. Terephthalic acid containing a radioactive carbon atom ¹⁴C.

10. The terephthalic acid according to claim 9, wherein the radioactive carbon atom ¹⁴C is derived from bio-naphtha.

11. A method for producing polyethylene terephthalate with a naphtha raw material containing renewable naphtha, the method comprising:
step (A-4) of obtaining terephthalic acid by the method according to claim 8; and
step (A-5) of obtaining polyethylene terephthalate by condensation reaction of terephthalic acid and ethylene glycol.

12. Polyethylene terephthalate containing a bio-naphtha-derived radioactive carbon atom ¹⁴C.

13. A management method for a monocyclic aromatic hydrocarbon to be used in production of at least one monocyclic aromatic hydrocarbon of benzene or xylene with a naphtha raw material containing renewable naphtha, wherein
the management method is a method for assigning a value as a renewable product, to the at least one monocyclic aromatic hydrocarbon, depending on a content rate of the renewable naphtha contained in the naphtha raw material,
the management method comprises at least one or more steps selected from step (V) of confirming that xylene and/or benzene are/is produced and step (W) of confirming that para-xylene is obtained,
the step (V) includes the following step (V-1), the following step (V-2), and the following step (V-3),
the step (V-1) is a step of confirming that toluene is produced from the naphtha raw material loaded to an apparatus for pyrolysis in the presence of water vapor (steam cracker),
the step (V-2) is a step of confirming that xylene and/or benzene are/is produced from toluene loaded to a disproportionation apparatus, or loaded to a transalkylation (TA) apparatus taken together with a C9-based component,
the step (V-3) is a step of confirming that xylene and/or benzene are/is produced from the naphtha raw material by treatment in the apparatus for pyrolysis in the presence of water vapor (steam cracker), and the disproportionation apparatus or the transalkylation (TA) apparatus in the listed order,
the step (W) includes the following step (V-1), the following step (V-2), the following step (W-3), and the following step (W-4),
the step (V-1) is a step of confirming that toluene is produced from the naphtha raw material loaded to an apparatus for pyrolysis in the presence of water vapor (steam cracker),
the step (V-2) is a step of confirming that xylene and/or benzene are/is produced from toluene loaded to a disproportionation apparatus, or loaded to a transalkylation (TA) apparatus taken together with a C9-based component,
the step (W-3) is a step of confirming that para-xylene is obtained from xylene loaded to a para-xylene apparatus,
the step (W-4) is a step of confirming that para-xylene is obtained from the naphtha raw material by treatment in the apparatus for pyrolysis in the presence of water vapor (steam cracker), the disproportionation apparatus or the transalkylation (TA) apparatus, and the para-xylene apparatus in the listed order,
the management method comprises step (Z) of confirming a proportion of a product to which a value as a renewable product is to be assigned, in a case which the management method comprises the step (V) and the step (W),
the step (Z) includes the following step (Z-1), the following step (Z-2), the following step (Z-3), and the following step (Z-4),
the step (Z-1) is a step of selecting one or more products to be assigned to renewable product(s), in products obtained by the disproportionation apparatus or the transalkylation (TA) apparatus, and the para-xylene apparatus,
the step (Z-2) is a step of determining a value of a proportion (P) of the product(s) to be assigned to renewable product(s) in a proportion of the product(s) selected in the step (Z-1), with respect to the products obtained by the disproportionation apparatus or the transalkylation (TA) apparatus, and the para-xylene apparatus,
the step (Z-3) is a step of grasping a value of a content rate (Q) of the renewable naphtha contained in the naphtha raw material, and
the step (Z-4) is a step of comparing a value of the proportion (P) and a value of the content rate (Q), and confirming that the value of the proportion (P) is equal to or less than the value of the content rate (Q).

14. The management method according to claim 13, wherein, in a case where the management method comprises the step (V) and does not comprise the step (W), the management method comprises step (X) of confirming a proportion of a product to which a value as a renewable product is to be assigned,
the step (X) includes the following step (X-1), the following step (X-2), the following step (X-3), and the following step (X-4),
the step (X-1) is a step of selecting one or more products to be assigned to renewable product(s), in products produced by the disproportionation apparatus or the transalkylation (TA) apparatus,
the step (X-2) is a step of determining a value of a proportion (P) of the product(s) to be assigned to renewable product(s) in a proportion of the products selected in the step (X-1), with respect to the products obtained by the disproportionation apparatus or the transalkylation (TA) apparatus,
the step (X-3) is a step of grasping a value of a content rate (Q) of the renewable naphtha contained in the naphtha raw material, and
the step (X-4) is a step of comparing a value of the proportion (P) and a value of the content rate (Q), and confirming that the value of the proportion (P) is equal to or less than the value of the content rate (Q).

15. The management method according to claim 13, wherein, in a case where the management method comprises the step (W) and does not comprise the step (V), the management method comprises step (Y) of confirming a proportion of a product to which a value as a renewable product is to be assigned,
the step (Y) includes the following step (Y-1), the following step (Y-2), the following step (Y-3), and the following step (Y-4),
the step (Y-1) is a step of selecting one or more products to be assigned to renewable product(s), in products produced by the para-xylene apparatus,
the step (Y-2) is a step of determining a value of a proportion (P) of the product(s) to be assigned to renewable product(s) in a proportion of the product(s) selected in the step (Y-1), with respect to the products obtained by the para-xylene apparatus,
the step (Y-3) is a step of grasping a value of a content rate (Q) of the renewable naphtha contained in the naphtha raw material, and
the step (Y-4) is a step of comparing a value of the proportion (P) and a value of the content rate (Q), and confirming that the value of the proportion (P) is equal to or less than the value of the content rate (Q).

16. The management method according to claim 15, wherein, in a case where two or more of the products to be assigned to renewable products are selected in the step (X-1), the step (Y-1) or the step (Z-1), the value of the proportion (P) of the products to be assigned to renewable products, determined in the step (X-2), the step (Y-2), or the step (Z-2) is a total value of respective proportions to be assinged to the two or more of the products selected.

17. The management method according to claim 13, wherein, in a case where the management method comprises the step (V) and the step (W), and two of the products to be assigned to renewable products, benzene and para-xylene, are selected in the step (Z-1), and
the value of the proportion (P) of the products to be assigned to renewable products, determined in the step (Z-2), is a total value of a proportion (P1) to be assigned to renewable benzene, in a proportion of benzene relative to the products obtained by the disproportionation apparatus or the transalkylation (TA) apparatus, and the para-xylene apparatus, and a proportion (P2) to be assigned to renewable para-xylene, in a proportion of para-xylene with respect to the products obtained by the disproportionation apparatus or the transalkylation (TA) apparatus, and the para-xylene apparatus.

18. A management method for terephthalic acid to be used in production of terephthalic acid with a naphtha raw material containing renewable naphtha, wherein
the management method is a method for assigning a value as a renewable product, to the terephthalic acid, depending on a content rate of the renewable naphtha contained in the naphtha raw material,
the management method comprises step (E) of confirming that terephthalic acid is obtained,
the step (E) includes the following step (V-1), the following step (G-2), the following step (W-3), the following step (E-4), and the following step (E-5),
the step (V-1) is a step of confirming that toluene is produced from the naphtha raw material loaded to an apparatus for pyrolysis in the presence of water vapor (steam cracker),
the step (G-2) is a step of confirming that xylene is produced from toluene loaded to a disproportionation apparatus, or loaded to a transalkylation (TA) apparatus taken together with a C9-based component ,
the step (W-3) is a step of confirming that para-xylene is obtained from xylene loaded to a para-xylene apparatus,
the step (E-4) is a step of confirming that terephthalic acid is obtained from para-xylene loaded to a terephthalic acid apparatus,
the step (E-5) is a step of confirming that terephthalic acid is obtained from the naphtha raw material by treatment in the apparatus for pyrolysis in the presence of water vapor (steam cracker), the disproportionation apparatus or the transalkylation (TA) apparatus, the para-xylene apparatus, and the terephthalic acid apparatus in the listed order,
the management method comprises step (H) of confirming a proportion of a product to which a value as a renewable product is to be assigned,
the step (H) includes the following step (H-1), the following step (H-2), the following step (H-3), and the following step (H-4),
the step (H-1) is a step of selecting a product to be assigned to a renewable product, in products obtained by the terephthalic acid apparatus,
the step (H-2) is a step of determining a value of a proportion (P) of the product to be assigned to a renewable product in a proportion of the product selected in step (H-1), with respect to the products obtained by the terephthalic acid apparatus,
the step (H-3) is a step of grasping a value of a content rate (Q) of the renewable naphtha contained in the naphtha raw material, and
the step (H-4) is a step of comparing a value of the proportion (P) and a value of the content rate (Q), and confirming that the value of the proportion (P) is equal to or less than the value of the content rate (Q).

19. A manegement method for polyethylene terephthalate to be used in production of polyethylene terephthalate with a naphtha raw material containing renewable naphtha, wherein
the management method is a method for assiging a value as a renewable product, to the polyethylene terephthalate, depending on a content rate of the renewable naphtha contained in the naphtha raw material,
the management method comprises step (F) of confirming that polyethylene terephthalate is obtained,
the step (F) includes the following step (V-1), the following step (G-2), the following step (W-3), the following step (E-4), the following step (F-5), and the following step (F-6),
the step (V-1) is a step of confirming that toluene is produced from the naphtha raw material loaded to an apparatus for pyrolysis in the presence of water vapor (steam cracker),
the step (G-2) is a step of confirming that xylene is produced from toluene loaded to a disproportionation apparatus, or loaded to a transalkylation (TA) apparatus taken together with a C9-based component,
the step (W-3) is a step of confirming that para-xylene is obtained from xylene loaded to a para-xylene apparatus,
the step (E-4) is a step of confirming that terephthalic acid is obtained from para-xylene loaded to a terephthalic acid apparatus,
the step (F-5) is a step of confirming that polyethylene terephthalate is obtained from terephthalic acid loaded to a PET apparatus,
the step (F-6) is a step of confirming that polyethylene terephthalate is obtained from the naphtha raw material by treatment in the apparatus for pyrolysis in the presence of water vapor (steam cracker), the disproportionation apparatus or the transalkylation (TA) apparatus, the para-xylene apparatus, the terephthalic acid apparatus, and the PET apparatus in the listed order,
the management method comprises step (J) of confirming a proportion of a product to which a value as a renewable product is to be assigned,
the step (J) includes the following step (J-1), the following step (J-2), the following step (J-3), and the following step (J-4),
the step (J-1) is a step of selecting a product to be assigned to a renewable product, in products obtained by the PET apparatus,
the step (J-2) is a step of determining a value of a proportion (P) of the product to be assigned to a renewable product in a proportion of the product selected in the step (J-1), with respect to the products obtained by the PET apparatus,
the step (J-3) is a step of grasping a value of a content rate (Q) of the renewable naphtha contained in the naphtha raw material, and
the step (J-4) is a step of comparing a value of the proportion (P) and a value of the content rate (Q), and confirming that the value of the proportion (P) is equal to or less than the value of the content rate (Q).

20. A management apparatus comprising a computer-readable storage medium in which a management program is stored, wherein
the management apparatus implements the management program, to implement the management method according to any one of claims 13 to 19.

21. The management apparatus according to claim 20, wherein, after the management method is implemented, a result which is acquired by the management method and to which a value as a renewable product to a product selected depending on a content rate of renewable naphtha contained in a naphtha raw material is assigned is output.

22. A computer-readable storage medium in which a computer program is stored, wherein
a management program which allows a computer to implement the management method according to any one of claims 13 to 19 is stored.

23. A management program for allowing a computer to implement the management method according to any one of claims 13 to 19.
